# FASCICULE DE BREVET EUROPEEN

(11) **EP 3 194 349 B1**
(45) Date de publication et mention de la délivrance du brevet: **07.11.2018**
(21) Numéro de dépôt: 15763370.2
(22) Date de dépôt: 17.09.2015
(51) Int. Cl.: C07C 1/20, C07C 11/167

(54) **PROCÉDÉ DE PRODUCTION DE BUTADIÈNE ET D'HYDROGÈNE À PARTIR D'ÉTHANOL EN DEUX ÉTAPES RÉACTIONNELLES À FAIBLE CONSOMMATION EN EAU ET EN ÉNERGIE**
VERFAHREN ZUR HERSTELLUNG VON BUTADIEN UND WASSERSTOFF AUS ETHANOL IN ZWEI REAKTIONSSCHRITTEN MIT GERINGEM WASSER- UND ENERGIEVERBRAUCH
METHOD FOR THE PRODUCTION OF BUTADIENE AND HYDROGEN FROM ETHANOL IN TWO LOW-WATER- AND LOW-ENERGY-CONSUMPTION REACTION STEPS

(30) Priorité: 19.09.2014 FR 1458859
(43) Date de publication de la demande: 26.07.2017
(73) Titulaire: IFP Energies nouvelles, 92500 Rueil-Malmaison (FR); Compagnie Générale des Etablissements Michelin, 63000 Clermont-Ferrand (FR)
(72) Inventeur: DASTILLUNG, Rejane, F-69005 Lyon (FR); FISCHER, Beatrice, F-69005 Lyon (FR); JACQUIN, Marc, F-69002 Lyon (FR); HUYGHE, Raphael, F-69700 Saint Andeol Le Chateau (FR)
(74) Mandataire: IFP Energies nouvelles
(86) Numéro de dépôt international: PCT/EP2015/071361
(87) Numéro de publication internationale: WO 2016/042095

(56) Documents cités:
- US-A- 2 403 742
- US-A- 2 403 743

## Description

### DOMAINE TECHNIQUE DE L'INVENTION

L'invention concerne un procédé de production de butadiène à partir d'éthanol opérant en deux étapes réactionnelles : une première étape réactionnelle produisant de l'acétaldéhyde, et une seconde étape produisant du butadiène à partir d'un mélange d'éthanol et d'acétaldéhyde.

### ART ANTÉRIEUR

Les procédés de production de butadiène à partir d'éthanol ont été développés, en particulier, par les Russes sur la base des travaux de Lebedev dans les années 20 (procédé en 1 étape réactionnelle), et par les Américains durant la seconde guerre mondiale à partir des travaux d'Ostromilenski (procédé en 2 étapes réactionnelles : déshydrogénation d'éthanol en acétaldéhyde, puis production du butadiène à partir d'un mélange éthanol/acétaldéhyde). Ce dernier procédé, qui permet des rendements un peu meilleurs, a été opéré pendant les années 40 aux États-Unis. Toutes les unités de ce type sont depuis longtemps arrêtées pour des raisons principalement économiques.

Le procédé, dans sa version Lebedev ou Ostromilenski, a une conversion par passe largement inférieure à 50%, ce qui implique des recyclages importants, et compliqués pour ajuster exactement le ratio éthanol/acétaldéhyde à l'entrée du deuxième réacteur.

Un autre problème du procédé est la production d'une grande variété d'impuretés de toutes sortes : des hydrocarbures saturés, insaturés, et aromatiques, mais aussi des produits oxygénés (tels que des alcools, des phénols, des aldéhydes, des cétones, des acides, des esters, des éthers, des acétals).

Certains de ces sous-produits, tant gazeux que liquides dans les conditions normales de température et de pression, sont générés en quantité significative. Parmi les sous-produits gazeux, on peut citer l'hydrogène, le monoxyde de carbone, le dioxyde de carbone, les alcanes et oléfines en C₁-C₄, le méthyle éthyle éther. Parmi les sous-produits liquides, on peut citer les pentènes, les pentadiènes, le diéthyle éther, l'éthyle vinyl éther, les hexènes, les hexadiènes, le butanal, le crotonaldéhyde, l'acétate d'éthyle, le diéthyle acétal, le butanol, l'hexanol, l'acide acétique. Ces sous-produits gazeux et liquides sont problématiques pour l'obtention d'un butadiène aux spécifications, mais aussi car leur recyclage vers les étapes réactionnelles avec l'éthanol et l'acétaldéhyde induit une diminution du rendement global de l'unité. Leur extraction complique grandement le procédé de séparation.

D'autres sous-produits sont générés en quantité infimes. Dans la suite du document, on parlera d'"huiles brunes" pour designer l'ensemble de milliers de composés hydrocarbures et oxygénés produits dans les sections réactionnelles dont les températures d'ébullition sont comprises entre celle de l'éthanol et allant jusqu'à 600°C. Ces huiles brunes ont la particularité d'être solubles dans l'éthanol, mais insolubles dans l'eau. Elles peuvent, partout où elles ne sont pas diluées par un fort excès d'éthanol, encrasser et boucher les équipements. Par ailleurs, ces huiles brunes sont problématiques au sein de la colonne à distiller qui sépare l'eau produite par la réaction et l'éthanol non converti. En effet, ces huiles brunes sont solubles dans l'effluent eau-éthanol alimentant ladite colonne à distiller, et insolubles dans le résidu essentiellement constitué d'eau. Une séparation de phase se produit donc au sein de cette colonne, diminuant considérablement l'efficacité de la séparation. Les huiles brunes sont difficiles à éliminer au sein du procédé du fait qu'elles sont constituées de milliers de composés ayant des propriétés physico-chimique très différentes. Une fraction de ces huiles brunes s'accumule donc au sein du procédé, entrainant une baisse de son efficacité au bout de quelques jours, au mieux semaines, d'opération et nécessitant de purger périodiquement certains flux. La perte en éthanol et en acétaldéhyde engendrée dégrade le rendement global du procédé pour un coût qui serait aujourd'hui rédhibitoire.

Du fait des nombreuses impuretés produites par le procédé, la purification du butadiène est complexe. Elle fait appel à une combinaison de nombreuses opérations unitaires, telles que des lavages, des distillations simples et extractives. L'art antérieur enseigne l'utilisation de distillations extractives mettent en oeuvre un solvant bis(2-chloroéthyle)éther (Chlorex), aujourd'hui proscrit du fait de sa forte toxicité. Il est important de noter que les spécifications du butadiène sont aujourd'hui extrêmement sévères, de par la sensibilité des catalyseurs de polymérisation du butadiène. La reproduction de l'enchainement des opérations unitaires selon l'art antérieur ne permettrait donc pas d'atteindre les spécifications actuelles. Par exemple, la spécification en acétaldéhyde dans le butadiène est passée de 1000 ppm à moins de 10 ppm aujourd'hui.

L'ouvrage « Synthetic rubber », chapitre 4 (W.J. Toussaint et J. Lee Marah) donne une vue générale du procédé développé par la société Carbide and Carbon, dont les principales étapes sont résumées ci-dessous.

L'éthanol issu d'une étape de traitement des effluents est converti en un effluent éthanol/acétaldéhyde dans une première étape réactionnelle. Cette étape produit également un effluent hydrogène gazeux. L'effluent éthanol/acétaldéhyde est ensuite traité dans une étape de traitement des effluents, en pouvant être au préalable envoyé vers une étape d'extraction du butadiène, de manière à séparer un effluent éthanol et un effluent acétaldéhyde. L'effluent éthanol/acétaldéhyde contient un quantité importante d'acétaldéhyde (environ 20% poids), qui est beaucoup plus volatile que l'éthanol, et donc plus facilement entrainé au sein de l'effluent gazeux.

L'effluent hydrogène est traité dans une étape de lavage à l'eau qui permet de récupérer l'éthanol et l'acétaldéhyde entrainés avec l'hydrogène. Un lavage efficace permettant de ne pas diminuer le rendement global de l'unité par une perte d'éthanol et d'acétaldéhyde implique d'utiliser au moins 2 tonne d'eau par tonne de butadiène produit ou un équivalent de l'ordre de 5 t d'eau par tonne d'acétaldéhyde et d'éthanol récupérés. L'eau de lavage doit ensuite être traitée dans une étape de traitement des effluents. La récupération des traces d'éthanol et d'acétaldéhyde entraîne donc une augmentation conséquente de la taille des équipements et de la consommation énergétique du procédé. L'hydrogène a une pureté de l'ordre de 99% molaire ou 90% poids.

La seconde étape réactionnelle de conversion d'un mélange éthanol/acétaldéhyde en butadiène est alimentée par de l'éthanol et de l'acétaldéhyde issus d'une étape de traitement des effluents. Elle produit un effluent liquide et un effluent gazeux. Le fait de séparer les produits issus de la première étape réactionnelle dans une étape de traitement des effluents pour les mélanger de nouveau en entrée de la seconde étape réactionnelle entraîne une augmentation de la taille des équipements et de la consommation énergétique du procédé.

Dans l'art antérieur, l'effluent gazeux issu de la seconde étape réactionnelle est lavé avec des effluents riche en éthanol mais impurs, ce qui a pour conséquence de devoir sur-dimensionner l'étape d'extraction du butadiène et l'étape de traitement des sous-produits gazeux, et donc par conséquent l'étape de traitement des effluents.

Les effluents de la seconde étape réactionnelle sont traités dans une étape d'extraction du butadiène, laquelle est également alimentée par une partie de l'effluent éthanol/acétaldéhyde issu de la première étape réactionnelle. Cette étape comprend au moins une section de lavage gaz-liquide et une section de distillation. Cette étape d'extraction produit un effluent butadiène brut, un effluent de produits gazeux, et un effluent éthanol/acétaldéhyde/eau. Ce dernier peut être recyclé en tête de la section de lavage gaz-liquide de l'étape. Cependant, ce recyclage est problématique, car la présence d'eau dans l'éthanol réduit la solubilité du butadiène. Il faut donc augmenter les débits liquides pour extraire la même quantité de butadiène du flux gazeux lavé afin de compenser la diminution de solubilité.

L'effluent butadiène brut est lavé à l'eau dans une étape de première purification puis purifié dans une ultime purification, entre autre par distillation extractive mettant en oeuvre un solvant de type bis(2-chloroéthyle)éther (Chlorex). L'eau issue du lavage du butadiène brut est traitée dans une étape de traitement des effluents, et les impuretés issues de l'ultime purification sont éliminées du procédé.

L'étape de première purification du butadiène permet d'éliminer l'acétaldéhyde présent dans l'effluent butadiène brut issu de l'étape d'extraction du butadiène. Néanmoins, compte tenu de l'évolution des spécifications en carbonyle dans le butadiène entre les années 60 et aujourd'hui, le débit d'eau alimentant l'étape de première purification du butadiène devrait être fortement augmenté pour atteindre les spécifications actuelles. L'effluent eau usée ainsi généré étant envoyé vers l'étape de traitement des effluents, l'atteinte des spécifications se traduirait par une augmentation importante de la taille des équipements et de la consommation énergétique. De plus, l'augmentation du débit d'eau de lavage pour atteindre la spécification se traduirait également par des problématiques de démixtion butadiène/eau dans la colonne de lavage.

L'étape de traitement des effluents permet de séparer, à partir des différents effluents traités, un effluent acétaldéhyde, un effluent éthanol, un effluent eau, un effluent de sous-produits liquides et éventuellement un effluent huiles brunes. Cette étape comprend généralement de deux à trois colonnes à distiller. La charge éthanol du procédé est alimentée dans l'étape de traitement des effluents au niveau de la distillation éthanol/eau.

L'effluent huiles brunes éventuellement produit dans cette étape est soit éliminé du procédé, soit traité dans une étape spécifique de traitement des sous-produits liquides et des huiles brunes permettant de produire différents effluents valorisables, comme un effluent riche en acétate d'éthyle ou encore un effluent riche en hexadiènes et visant à minimiser les pertes en éthanol et acétaldéhyde avec les sous-produits liquides et les huiles brunes.

La complexité des recyclages entre les différentes opérations unitaires de l'étape de traitement des effluents rend cette étape délicate à opérer. Par ailleurs, les séparations de phase qui se produisent au sein des colonnes, notamment à cause des huiles brunes sont problématiques pour l'opération, mais aussi pour le dimensionnement des équipements.

Les sous-produits gazeux sont traités dans une étape de traitement des sous-produits gazeux dans laquelle ils sont lavés par un flux d'eau, lequel est ensuite traité dans l'étape de traitement des effluents.

Le brevet US 1,977,750 décrit les étapes de conversion de l'éthanol en acétaldéhyde et de traitement de l'hydrogène. L'éthanol est partiellement converti dans une section catalytique. L'effluent, constitué principalement d'éthanol, d'acétaldéhyde et d'hydrogène, est refroidi dans un condenseur à eau, puis dans un condenseur alimenté par une saumure, avant d'être lavé à l'eau. L'ensemble des effluents liquides collectés dans ces trois opérations unitaires sont rassemblés et distillés afin de récupérer l'acétaldéhyde et l'éthanol non converti.

Le brevet US 2,249,847 décrit les étapes de conversion de l'éthanol en acétaldéhyde, et de traitement des effluents, dans la cas d'un procédé ne produisant que de l'acétaldéhyde et ses sous-produits. En particulier, il décrit l'intérêt d'introduire la charge éthanol dans le ballon de reflux de la colonne à distiller éthanol/eau de l'étape de traitement des effluents, pratique reproduite dans l'ensemble des documents postérieurs.

Le brevet US 2,403,741 décrit les étapes de conversion de l'éthanol en acétaldéhyde, de conversion du mélange éthanol/acétaldéhyde en butadiène, de traitement de l'hydrogène, d'extraction du butadiène, de première purification du butadiène, et de traitement des effluents. Il décrit l'intérêt d'utiliser l'effluent éthanol/acétaldéhyde issu de la première étape réactionnelle pour réaliser l'étape d'extraction du butadiène compris dans l'effluent vapeur issu de la seconde étape réactionnelle.

Le brevet US 2,403,742 décrit les étapes de conversion de l'éthanol en acétaldéhyde, de conversion du mélange éthanol/acétaldéhyde en butadiène, de traitement de l'hydrogène, d'extraction du butadiène, de première purification du butadiène, et de traitement des effluents. Il décrit la difficulté de recycler un effluent acétaldéhyde issu de l'étape de traitement des effluents vers la seconde étape réactionnelle, notamment à cause de la présence de diéthyle éther, qui forme un azéotrope avec l'acétaldéhyde. L'étape de traitement des effluents met donc en oeuvre une colonne à distiller intermédiaire entre celle produisant l'acétaldéhyde et celle produisant l'éthanol afin d'éliminer les sous-produits liquides. Cette pratique se retrouve dans l'ensemble des documents postérieurs.

Les brevets US 2,403,743, US 2,393,381 et 2,395,057 décrivent différentes configurations de l'étape de traitement des effluents et la présence éventuelle d'une étape de traitement des sous-produits liquides et huiles brunes afin de minimiser les pertes en éthanol et acétaldéhyde. Ces documents nous apprennent que sans réaliser les inventions décrites, la pureté de l'effluent acétaldéhyde issu de l'étape de traitement des effluents peut descendre en dessous de 50% pds, entraînant de graves problèmes lorsque ce dernier est recyclé vers la seconde étape réactionnelle. Les inventions proposées permettent d'obtenir un effluent acétaldéhyde/ethanol issu de l'étape de traitement des effluents ayant une pureté supérieure à 80% pds, et dans le meilleur des cas égale à 93%pds. Néanmoins les trains de séparation décrits sont de toute évidence difficiles à opérer et énergivores, compte tenu des nombreux recyclages entre les différentes opérations unitaires.

Le brevet US 2,409,250 (Carbide and Carbon, 1944) décrit les étapes de purification successives du butadiène (extraction, première purification et ultime purification du butadiène par super-fractionnement). Le butadiène est produit à une pureté de 98,7%, mais au prix d'une perte significative de rendement. Pour limiter cette perte, les produits de têtes de la colonnes de purification du butadiène par super-fractionnement sont soutirés et en partie recyclés vers l'étape d'extraction du butadiène. Ces recyclages importants, en particulier le recyclage du flux butène/butadiène en vue d'éliminer les incondensables, induisent un surdimensionnement des équipements.

La société Koppers propose, dans le brevet US 2,439,587, une amélioration du procédé Carbide et Carbon consistant à récupérer le sous-produit acétate d'éthyle et à le recycler dans la seconde étape réactionnelle. Les opérations de séparation sont complexes et difficiles à régler, car elles mettent en oeuvre entre autre des soutirages latéraux, des décantations, des purges, des lavages à l'eau, et induisent des pertes importantes en éthanol et en acétaldéhyde.

Le brevet US 1,948,777 (Carbide and Carbon, 1931) décrit dans le détail l'étape d'ultime purification du butadiène par distillation extractive mettant en oeuvre différents solvants, dont le Chlorex. En limitant la perte en butadiène en tête de colonne, soit une concentration de 0,2% de butadiène dans la distillat, la pureté du butadiène obtenu en fond n'est que de 70%, alors qu'en cherchant à obtenir un butadiène plus pur en fond, soit 99%, la perte en butadiène en tête est beaucoup plus important, avec une concentration de butadiène dans le distillat de 30%. La production d'un butadiène de haute pureté est donc réalisée au prix d'une baisse de rendement global de l'unité.

Le rendement global des schémas de l'art antérieur est faible, étant donné que l'effluent acétaldéhyde recyclé vers la seconde étape réactionnel est impur, et compte tenu des pertes en éthanol et acétaldéhyde.

Même si actuellement la principale source de butadiène est pétrolière, la raréfaction future des gisements conduit à repenser entièrement ce procédé ancien, afin de permettre la production de butadiène à partir d'une ressource alternative.

### OBJET ET INTÉRÊT DE L'INVENTION

L'invention a pour objet un procédé de production de butadiène à partir d'une charge éthanol comprenant au moins 80% poids d'éthanol comprenant au moins :
A) une étape de conversion de l'éthanol en acétaldéhyde comprenant au moins une section réactionnelle alimentée au moins par une fraction de l'effluent riche en éthanol issu de l'étape E1), opérée à une pression comprise entre 0,1 et 1,0 MPa et à une température comprise entre 200 et 500°C en présence d'un catalyseur, et une section de séparation permettant de séparer l'effluent de ladite section réactionnelle en au moins un effluent hydrogène sous forme gazeuse et un effluent éthanol/acétaldéhyde sous forme liquide ;
B) une étape de conversion en butadiène comprenant au moins une section réactionnelle alimentée au moins par une fraction dudit effluent éthanol/acétaldéhyde issu de l'étape A), par un effluent liquide riche en éthanol issu de l'étape C1), par une fraction de l'effluent riche en acétaldéhyde issu de l'étape E1), opérée en présence d'un catalyseur, à une température comprise entre 300 et 400°C, et à une pression comprise entre 0,1 et 1,0 MPa, les débits d'alimentation étant réglés de telle sorte que le rapport molaire éthanol/acétaldéhyde en entrée de ladite section réactionnelle est compris entre 1 et 5, et une section de séparation permettant de séparer l'effluent de ladite section réactionnelle en au moins un effluent gazeux et un effluent liquide ;
C1) une étape de traitement de l'hydrogène comprenant au moins une section de compression comprimant ledit effluent hydrogène issu de l'étape A) à une pression comprise entre 0,1 et 1,0 MPa, et une section de lavage gaz-liquide alimentée à une température comprise entre 15°C et -30°C par une fraction dudit effluent éthanol issu de l'étape E1) et par une fraction dudit effluent éthanol/acétaldéhyde issu de l'étape A), et alimentée à une température comprise entre 25 et 60°C par ledit effluent hydrogène comprimé, et produisant au moins un effluent liquide riche en éthanol et un effluent hydrogène purifié ;
D1) une étape d'extraction du butadiène comprenant au moins une section de compression comprimant ledit effluent gazeux issu de l'étape B) à une pression comprise entre 0,1 et 1,0 MPa, une section de lavage gaz-liquide comprenant une colonne de lavage alimentée en tête à une température comprise entre 20 et -20°C par un flux éthanol constitué de ladite charge éthanol du procédé et/ou d'une fraction de l'effluent éthanol issu de l'étape E1) et en fond par ledit effluent gazeux issue de l'étape B) et refroidi, et une section de distillation opérée à une pression comprise entre 0,1 et 1 MPa, alimentée au moins par l'effluent liquide issu de ladite étape B) et par l'effluent liquide de ladite section de lavage gaz-liquide, ladite étape D1) produisant au moins un effluent sous-produits gazeux, un effluent butadiène brut, et un effluent éthanol / acétaldéhyde / eau;
D2) une étape de première purification du butadiène comprenant au moins une section de lavage gaz-liquide alimentée en fond par l'effluent butadiène brut issu de D1) et en tête par un flux d'eau pouvant être un flux d'eau d'origine externe audit procédé de production de butadiène et/ou une fraction de l'effluent eau issu de l'étape E1), ladite section de lavage produisant en tête un effluent butadiène pré-purifié et en fond un effluent eau usée ;
D3) une étape ultérieure de purification du butadiène, alimentée au moins par ledit effluent butadiène pré-purifié issu de ladite étape D2), et produisant au moins un effluent butadiène purifié ;
E1) une étape traitement des effluents alimentée au moins par le raffinat eau / éthanol / acétaldéhyde issu de l'étape E2), et produisant au moins un effluent riche en éthanol, un effluent riche en acétaldéhyde et un effluent riche en eau ;
E2) une étape d'élimination des impuretés et des huiles brunes, alimentée au moins par l'effluent éthanol/acétaldéhyde/eau issu de l'étape D1), et par l'effluent riche en eau issu de l'étape E1), et produisant au moins un raffinat eau / éthanol / acétaldéhyde, un effluent huiles brunes légères et un effluent huiles brunes lourdes ;
F) une étape de lavage à l'eau, alimentée par l'effluent sous-produits gazeux issu de l'étape D1), ainsi que par une fraction de l'effluent riche en eau issu de ladite étape E1) et produisant au moins un effluent eau alcoolisée.

La demanderesse a identifié un agencement d'opérations unitaires, qui permettent de palier à de nombreux inconvénients de l'art antérieur. En particulier, l'agencement des opérations unitaires selon l'invention permet de d'éliminer les impuretés gazeuses, les impuretés liquides et les huiles brunes tout en minimisant la perte en éthanol et acétaldéhyde, améliorant ainsi le rendement global de l'unité tout en réduisant le flux global d'eau nécessaire aux étapes de séparation et en obtenant un butadiène très pur. La réduction importante du flux global d'eau permet de réduire la consommation énergétique du procédé et la taille des équipements de séparation.

### DESCRIPTION DÉTAILLÉE DE L'INVENTION

### Charge

La charge éthanol utilisée dans le procédé selon l'invention peut provenir de toute origine, fossile, végétale ou animale, et en particulier des procédés de production d'éthanol à partir de ressources végétale. Ladite charge comprend au moins 80% poids d'éthanol, préférentiellement au moins 90% poids, et de manière préférée au moins 93 % poids. De manière très préférée, ladite charge éthanol répond aux spécifications d'éthanol carburant EN 15376.

### Étape A) de conversion de l'éthanol en acétaldéhyde

Conformément à l'invention, une étape A) de conversion de l'éthanol en acétaldéhyde comprend au moins une section réactionnelle alimentée au moins par une fraction de l'effluent riche en éthanol issu de l'étape E1), ladite fraction constituant de préférence au moins 10% du débit dudit effluent riche en éthanol issu de l'étape E1), et optionnellement avantageusement alimentée par au moins une fraction de ladite charge éthanol, et une section de séparation permettant de séparer l'effluent de ladite section réactionnelle en au moins un effluent hydrogène sous forme gazeuse et un effluent éthanol/acétaldéhyde sous forme liquide.

Ladite section réactionnelle permet de convertir l'éthanol en acétaldéhyde en présence d'un catalyseur consistant de préférence en un mélange d'oxyde de chrome et d'oxyde de cuivre, ou de tout autre catalyseur adapté. Ces catalyseurs sont bien connus de l'Homme du métier.

Ladite section réactionnelle est opérée à une pression comprise entre 0,1 et 1,0 MPa, de préférence entre 0,1 et 0,5 MPa, de manière préférée entre 0,1 et 0,3 MPa, et à une température comprise entre 200 et 500°C, préférentiellement comprise entre 250 et 300°C.

De préférence, la conversion de l'éthanol est comprise entre 30 et 40%, avec une sélectivité comprise entre 85 et 100% vers l'acétaldéhyde, de manière préférée comprise entre 90 et 95% vers l'acétaldéhyde. L'effluent de ladite section réactionnelle comprend également des sous-produits tels que le crotonaldéhyde, butyraldehyde, le diethylacetal, l'éthyl acétate et l'acide acétique.

Ladite section de séparation met en oeuvre des moyens de séparation gaz-liquide connus de l'Homme du métier. De manière préférée, on utilisera un séparateur gaz-liquide opéré à une pression comprise entre 0,1 et 0,3 MPa, et une température comprise entre 25 et 60°C.

### Étape B) de conversion d'un mélange éthanol/acétaldéhyde en butadiène

Conformément à l'invention, une étape B) de conversion en butadiène comprend au moins une section réactionnelle alimentée au moins par une fraction dudit effluent éthanol/acétaldéhyde issu de l'étape A), par un effluent liquide riche en éthanol issu de l'étape C1), par une fraction de l'effluent riche en acétaldéhyde issu de l'étape E1) et optionnellement avantageusement alimentée par un flux riche en éthanol issu de l'étape E1), et une section de séparation permettant de séparer l'effluent de ladite section réactionnelle en au moins un effluent gazeux et un effluent liquide. Ladite section réactionnelle peut également être alimentée par un flux externe d'acétaldéhyde.

Le débit des différentes alimentations de la section réactionnelle de ladite étape B) est ajusté de telle sorte que le rapport molaire éthanol sur acétaldéhyde en entrée de ladite section réactionnelle est compris entre 1 et 5, de manière préférée compris entre 1 et 3,5, de manière encore plus préférée entre 2 et 3 et de manière très préférée entre 2,4 et 2,7.

Ladite section réactionnelle permet de convertir une partie du mélange éthanol/acétaldéhyde en au moins du butadiène. La sélectivité de la transformation du mélange éthanol/acétaldéhyde est de préférence supérieure à 60%, de manière préférée supérieure à 70%, de manière très préférée supérieure à 80%. Par sélectivité, on entend le rapport molaire du débit de butadiène dans l'effluent de ladite section réactionnelle sur le débit d'éthanol et d'acétaldéhyde consommé dans ladite section réactionnelle. La conversion de la transformation du mélange éthanol/acétaldéhyde est de préférence supérieure à 30%, de manière préférée supérieure à 40%, de manière préférée supérieure à 47%. Par conversion, on entend le rapport molaire du débit d'éthanol et d'acétaldéhyde dans l'effluent de ladite section réactionnelle sur le débit d'éthanol et d'acétaldéhyde dans l'alimentation de ladite section réactionnelle. Elle est opérée en présence d'un catalyseur, avantageusement un catalyseur supporté sur silice choisi dans le groupe constitué par les catalyseurs comprenant de l'oxyde de tantale, de zirconium ou de colombium, préférentiellement comprenant 2% d'oxyde de Tantale (voir par exemple Corson, Jones, Welling, Hincbley, Stahly, Ind. Eng Chem. 1950, 42, 2, 359-373). Ladite section réactionnelle est opérée à une température comprise entre 300 et 400°C, de manière préférée entre 320 et 370°C et à une pression comprise entre 0,1 et 1,0 MPa, de préférence entre 0,1 et 0,5 MPa, de manière préférée entre 0,1 et 0,3 MPa.

De préférence, environ 65 à 80% de l'acétaldéhyde est converti dans ladite section réactionnelle. L'effluent de ladite section réactionnelle comprend donc encore de l'éthanol. De nombreuses impuretés peuvent être produites avec le butadiène, parmi lesquelles de l'éthylène, du propylène, du diéthyl ether (DEE), de l'acétate d'éthyle, du butanol, de l'hexanol, des butènes, des pentènes, des pentadiènes, des hexènes, et des hexadiènes.

Ladite section réactionnelle étant alimentée par l'effluent acétaldéhyde issu de l'étape E1) de traitement des effluents, le ratio éthanol sur acétaldéhyde à l'entrée cette section est ajusté en contrôlant la fraction de l'effluent éthanol issu de ladite étape E1) alimentant l'étape A), et donc produisant de l'acétaldéhyde. En effet, la fraction restante de l'effluent riche en éthanol issu de ladite étape E1) alimente l'étape C1) de traitement de l'hydrogène et forme, après lavage de l'effluent hydrogène, ledit effluent liquide riche en éthanol issu de ladite étape C1) qui alimente ladite étape B). Or, cet effluent liquide riche en éthanol issu de ladite étape C1) ne contient que très peu d'acétaldéhyde. Le contrôle du ratio éthanol sur acétaldéhyde à l'entrée de ladite section réactionnelle est donc aisé avec le procédé selon l'invention.

Ladite section de séparation met en oeuvre des moyens de séparation gaz-liquide connus de l'Homme du métier. De manière préférée, on utilisera un séparateur gaz-liquide opéré à une pression comprise entre 0,1 et 0,3 MPa, et une température comprise entre 25 et 60°C.

### Étape C1) de traitement de l'hydrogène

Conformément à l'invention, une étape C1) de traitement de l'hydrogène comprend au moins une section de compression alimentée par ledit effluent hydrogène issu de l'étape A) et une section de lavage gaz-liquide alimentée par une fraction dudit effluent éthanol issu de ladite étape E1), et par une fraction dudit effluent éthanol/acétaldéhyde issu de ladite étape A), et produit au moins un effluent liquide riche en éthanol et un effluent hydrogène purifié. De préférence, ladite étape C1) n'est alimentée par aucun autre flux.

Ladite fraction dudit effluent éthanol/acétaldéhyde issu de l'étape A) est comprise entre 0 et 100%. L'utilisation d'une fraction dudit effluent éthanol/acétaldéhyde permet de diminuer le débit de la fraction dudit effluent riche en éthanol issu de ladite étape E1).

Ladite étape C1) permet d'obtenir un effluent hydrogène purifié très pur, c'est-à-dire comprenant au moins 90% molaire d'hydrogène, de préférence 99% molaire d'hydrogène, de préférence 99,8% molaire d'hydrogène. L'effluent hydrogène purifié comprend également des traces d'eau et d'éthanol. Cette étape permet également de récupérer l'éthanol et l'acétaldéhyde compris dans l'effluent hydrogène issu de ladite étape A), permettant ainsi leur recyclage et maximisant le rendement global du procédé.

L'utilisation d'une fraction de l'effluent riche en éthanol issu de l'étape E1) et d'une fraction de l'effluent éthanol/acétaldéhyde issu de l'étape A) en lieu et place d'eau - comme cela était réalisé dans l'art antérieur - permet de diminuer le débit total d'eau circulant dans le procédé. Ainsi, le débit total d'eau alimentant les étapes E1) et E2) de traitement des effluents est diminué, diminuant de ce fait la taille des équipements et la consommation en utilités des étapes E1) et E2). De plus, comme cela a été décrit précédemment, l'effluent liquide riche en éthanol issu de ladite étape C1) peut alimenter directement l'étape B) de conversion en butadiène sans avoir à être traité par l'étape E1) de traitement des effluents.

La demanderesse a découvert que, dans le procédé selon l'invention, l'éthanol est un bien meilleur solvant que l'eau pour abattre l'acétaldéhyde, ce qui permet de réduire la taille des équipements mis en oeuvre dans l'étape C1) selon l'invention par rapport à l'art antérieur.

L'effluent hydrogène issu de l'étape A) est comprimé dans une section de compression à une pression comprise entre 0,1 et 1,0 MPa, avantageusement entre 0,1 et 0,7 MPa, et de manière préférée entre 0,4 et 0,68 MPa. L'effet de cette compression est d'une part de diminuer le débit volumique de gaz, et d'autre part d'améliorer l'efficacité du lavage en aval.

L'effluent hydrogène comprimé est ensuite refroidi à une température comprise entre 25 et 60°C, préférentiellement entre 30°C et 40°C, puis alimente en fond la colonne de lavage de la section de lavage dans laquelle il est mis en contact avec ladite fraction de l'effluent éthanol issu de l'étape E1) et ladite fraction de l'effluent éthanol/acétaldéhyde issu de l'étape A), lesdites fractions étant respectivement alimentées en tête et en un point intermédiaire de ladite colonne de lavage. Ces deux fractions sont refroidies chacune à une température comprise entre 15°C et -30°C, préférentiellement entre 0°C et -15°C avant d'être alimentées dans ladite colonne de lavage. Avantageusement, l'effluent riche en éthanol issu de l'étape E1) sera alimenté à une température inférieure à celle de ladite fraction de l'effluent éthanol/acétaldéhyde issu de l'étape A), créant ainsi un gradient thermique entre la tête et le fond et limitant les pertes en solvant dans l'effluent hydrogène purifié. La colonne de lavage gaz-liquide de la section de lavage est munie de plateaux ou de garnissage vrac ou structurés.

La demanderesse a découvert que la présence d'une forte quantité d'éthanol par rapport à l'eau dans ladite section de lavage permet de fonctionner à de basses températures sans risque de former des hydrates dans cette colonne. La température basse des liquides de lavage et le gradient de température entre la tête et le fond permettent d'obtenir une très bonne récupération de l'acétaldéhyde présent dans l'effluent hydrogène comprimé, et une très bonne pureté de l'effluent hydrogène purifié, soutiré en tête de ladite section de lavage. Ainsi, la perte en acétaldéhyde dans l'effluent hydrogène purifié est nulle. De plus, la perte en éthanol dans l'hydrogène purifié est très faible du fait de la faible température de l'éthanol introduit en tête de la section de lavage, qui est autorisée par l'absence de formation d'hydrates.

### Étape optionnelle C2) de traitement final de l'effluent hydrogène

Une étape C2) de traitement final de l'hydrogène est avantageusement réalisée à l'issue de l'étape C1). Ladite étape C2) comprend au moins d'une section de lavage gaz-liquide, alimentée par l'effluent hydrogène purifié issu de C1), et par un effluent eau pure d'origine externe au procédé ou par un effluent riche en eau issu de l'étape E1), et produit un effluent hydrogène purifié et un effluent eau usé.

Ladite section de lavage comprend au moins une colonne de lavage gaz-liquide, alimentée en fond par ledit effluent hydrogène purifié issu de C1), et en tête par un effluent eau pure ou par un effluent riche en eau issu de l'étape E1) et produisant un effluent hydrogène purifié en tête et un effluent eau usé en fond.

Cette étape permet de récupérer les dernières traces d'éthanol éventuellement contenues dans l'effluent hydrogène purifié issu de C1). Ladite étape C2), similaire au traitement de l'hydrogène selon l'art antérieur, met néanmoins en oeuvre des débits d'eau bien inférieurs à ceux utilisés dans l'art antérieur, l'effluent hydrogène alimentant l'étape C2) ayant été traité au préalable par l'étape C1), et donc débarrassé de la totalité de l'acétaldéhyde entrainé dans l'effluent hydrogène issu de l'étape A). Toutes choses égales par ailleurs, l'élimination de traces d'éthanol par un lavage à l'eau demande des débits moindres que l'élimination de traces d'acétaldéhyde. De plus, l'éthanol étant moins volatil que l'acétaldéhyde, il est, dans les même conditions opératoires, beaucoup moins entrainé que l'acétaldéhyde.

### Étape D1) d'extraction du butadiène

Conformément à l'invention, une étape D1) d'extraction du butadiène comprenant au moins une section de compression, une section de lavage gaz-liquide, et une section de distillation est alimentée au moins par lesdits effluents gazeux et liquide issus de ladite étape B), par un flux éthanol constitué de ladite charge éthanol du procédé et/ou d'une fraction de l'effluent éthanol issu de l'étape E1), et produit au moins un effluent sous-produits gazeux, un effluent butadiène brut, et un effluent éthanol/acétaldéhyde/eau.

Ledit flux éthanol alimentant l'étape D1) comprend de préférence au moins 80% poids d'éthanol, préférentiellement au moins 90% poids, et de manière préférée au moins 93 % poids. Ledit flux éthanol alimentant l'étape D1) peut contenir du méthanol, de l'eau, de l'acétate d'éthyle, du butanol et de l'hexanol. De préférence, ledit flux éthanol alimentant l'étape D1) comprend moins de 10% poids d'acétaldéhyde, de préférence moins de 5% poids, et de manière préférée moins de 1% poids. De préférence, ledit flux éthanol alimentant l'étape D1) comprend moins de 20% poids d'eau, de préférence moins de 5% poids, de manière préférée moins de 1% poids.

Dans un arrangement préféré, ledit flux éthanol alimentant l'étape D1) est constitué de ladite charge éthanol du procédé. Un avantage de cet arrangement est que ladite charge est exempte des sous-produits des réactions qui sont formés dans les étapes A) et B) et qui peuvent se trouver concentrés par le biais des recyclages. En particulier, cette charge éthanol ne contient pas d'acétaldéhyde, ou seulement à l'état de traces.

Dans un autre arrangement préféré, ledit flux éthanol est constitué d'une fraction de l'effluent éthanol issu de l'étape E1) de traitement des effluents.

L'utilisation d'un flux éthanol contenant peu ou pas d'acétaldéhyde minimise l'entraînement d'acétaldéhyde dans ledit effluent sous-produits gazeux soutiré en tête de ladite section de lavage gaz-liquide réduisant les pertes en rendement global du procédé, ainsi que le débit d'eau de lavage nécessaire dans l'étape F).

L'effluent gazeux issu de l'étape B) est comprimé dans ladite section de compression à une pression comprise entre 0,1 et 1,0 MPa, préférentiellement entre 0,1 et 0,7 MPa, et de manière préférée entre 0,2 et 0,5 MPa. L'effet de cette compression est d'une part de diminuer le débit volumique de gaz, et d'autre part d'améliorer l'efficacité du lavage en aval. De préférence, l'effluent gazeux comprimé est ensuite refroidi à une température comprise entre 25 et 60°C, préférentiellement entre 30°C et 40°C.

De préférence, ladite section de lavage gaz-liquide de l'étape D1) comprend une colonne de lavage alimentée en tête par ledit flux éthanol alimentant l'étape D1), en fond par ledit effluent gazeux comprimé et refroidi et produit en tête l'effluent sous-produits gazeux et en fond un effluent liquide qui alimente ladite section de distillation de l'étape D1).

Ledit flux éthanol alimentant l'étape D1) est refroidi avant d'être alimenté en tête de ladite colonne de lavage gaz-liquide de la section de lavage à une température comprise entre 20 et -20°C, préférentiellement entre 15°C et 5°C. L'intérêt de refroidir ledit flux éthanol est d'améliorer la performance de l'opération de lavage en minimisant l'entraînement d'éthanol et d'acétaldéhyde dans ledit effluent sous-produits gazeux. Ainsi, la totalité du butadiène présent dans l'effluent gazeux issu de l'étape B) comprimé et refroidi est abattue, et l'effluent sous-produits vapeur soutiré en tête de ladite section de lavage gaz-liquide est exempt de butadiène.

La minimisation de l'entraînement d'acétaldéhyde dans ledit effluent sous-produits gazeux permet, incidemment, de diminuer de manière importante le débit d'eau requis dans l'étape F) de lavage à l'eau des sous-produits gazeux, dont l'objectif est de récupérer l'éthanol et les traces éventuelles d'acétaldéhyde entraînés dans l'effluent sous-produits gazeux soutiré en tête de la section de lavage à l'éthanol de l'étape D1).

De préférence, le flux éthanol enrichi en butadiène soutiré en fond de ladite section de lavage gaz-liquide de l'étape D1) ainsi que l'effluent liquide issu de l'étape B) alimentent ladite section de distillation de l'étape D1) de manière à séparer en tête un effluent vapeur comprenant la majorité du butadiène, appelé effluent butadiène brut, et en fond un résidu éthanol/acétaldéhyde/eau. Par la majorité, on entend plus de 80% du butadiène compris dans l'alimentation de ladite section de distillation, préférentiellement plus de 90%, de manière préférée plus de 95%, de manière encore plus préférée plus de 98%, de manière très préférée plus de 99% et de manière très avantageuse la totalité du butadiène compris dans ladite alimentation. Ce résidu éthanol/acétaldéhyde/eau comprend de l'éthanol et de l'acétaldéhyde, et comprend également de l'eau produite dans l'étape B) et des sous-produits formés dans les étapes A) et B), comme par exemple le diéthyléther et l'acétate d'éthyle et les huiles brunes. Ledit résidu éthanol/acétaldéhyde/eau alimente ensuite l'étape E2) de traitement des effluents. Ladite section de distillation est opérée à une pression comprise entre 0,1 et 1 MPa et de manière préférée entre 0,2 et 0,5 MPa.

L'agencement des recyclages et l'utilisation des flux externes (charge éthanol, eau) selon l'invention, et en particulier d'un flux éthanol refroidi, permet de minimiser le débit de l'effluent eau usée, et donc le débit à traiter par lesdites sections E1 et E2). Le procédé selon l'invention permet donc de minimiser le débit des effluents à traiter dans l'étape de traitement des effluents.

### Etape D2) de première purification du butadiène

L'étape D2) de première purification du butadiène comprend au moins une section de lavage gaz-liquide alimentée en fond par l'effluent butadiène brut issu de D1) et en tête par un flux d'eau pouvant être un flux d'eau d'origine externe audit procédé de production de butadiène et/ou une fraction de l'effluent eau issu de l'étape E1), ladite section de lavage produisant en tête un effluent butadiène pré-purifié et en fond un effluent eau usée. De manière préférée, ledit flux eau est un flux d'eau d'origine externe au procédé.

Ledit effluent eau usée contient de l'acétaldéhyde et un peu de butadiène, et peut être envoyé vers l'étape E1) de traitement des effluents, vers la section de distillation de l'acétaldéhyde, ou vers l'étape E2).

L'objectif de l'étape D2) est d'éliminer les impuretés polaires, en particulier l'acétaldéhyde qui ne doit pas être présent au-delà de quelques ppm dans le butadiène final. L'effluent butadiène brut issu de D1) comprend la majorité du butadiène, mais contient encore de nombreuses impuretés, dont une quantité importante d'acétaldéhyde qui forme un azéotrope avec le butadiène et ne peut donc pas être complètement éliminé par distillation lors de l'étape D1). Ainsi, le débit dudit flux d'eau est ajusté pour obtenir la spécification recherchée en acétaldéhyde dans l'effluent butadiène pré-purifié.

Ledit flux d'eau est refroidi à une température inférieure à 25°C, de préférence inférieure à 20°C avant d'alimenter la section de lavage gaz-liquide de manière à faire le lavage avec une quantité d'eau réduite. La température d'alimentation dudit flux d'eau est choisie de manière à ne pas former d'hydrates avec le butadiène et les hydrocarbures légers encore présents dans le flux butadiène brut issu de l'étape D1). La pression de la colonne de lavage est déterminée de façon à assurer qu'il n'y ait aucune condensation du butadiène et qu'il reste bien sous forme gaz. La pression sur cette étape est comprise entre 0,1 et 1 MPa, et de manière préférée entre 0,2 et 0,3 MPa.

### Étape D2bis) optionnelle de seconde purification du butadiène

L'effluent butadiène pré-purifié issu de l'étape D2) subit avantageusement une étape D2bis) de seconde purification du butadiène avant d'être alimentée dans l'étape D3) ultérieure de purification du butadiène, ladite étape D2bis) comprenant au moins une section de lavage alimentée en fond par ledit effluent butadiène pré-purifié issu de D2), et en tête par une solution absorbante. On soutire en tête de ladite section de lavage un effluent butadiène pré-purifié dans lequel ont été éliminées les traces d'acétaldéhyde encore contenues dans l'effluent butadiène pré-purifié, ainsi que les traces d'autres carbonyles qui sont moins solubles dans l'eau que l'acétaldéhyde, comme par exemple le butanal, l'acétone et l'hexanal, et donc moins efficacement éliminés par un simple lavage à l'eau. On soutire en fond de ladite section de lavage un effluent liquide qui est éliminé du procédé.

Dans un premier mode de réalisation de ladite étape D2bis), ladite solution absorbante est une solution aqueuse ayant un pH supérieur à 10, ajusté par l'ajout de soude ou de potasse.

Dans un deuxième mode de réalisation de ladite étape D2bis), ladite solution absorbante est une solution aqueuse de bisulfite de sodium ou de potassium dont le pH est compris entre 5 et 8, de manière préféré entre 6 et 7.

Dans un troisième mode de réalisation de ladite étape D2bis), ladite solution absorbante est une solution aqueuse contenant un composé de la famille des hydrazines.

La demanderesse a découvert que la combinaison des étapes D2) et D2bis) était particulièrement bien adaptée au traitement d'un effluent butadiène brut issu d'un procédé de production de butadiène à partir d'éthanol.

En effet, la mise en oeuvre de l'étape D2) seule nécessite de gros débits d'eau pour atteindre la spécification de moins de 10 ppm de composés carbonylés. Ces gros débits d'eau sont ensuite traités à l'étape E1), ce qui induit des coûts d'opération et d'investissement conséquents. De plus, en augmentant de manière très importante les débits d'eau alimentant l'étape D2), on solubilise une faible portion de butadiène, ce qui diminue le rendement global du procédé.

Par ailleurs, la mise en oeuvre de l'étape D2bis) seule ne conviendrait pas pour traiter un effluent butadiène brut issu de l'étape D1). En effet, l'acétaldéhyde éliminé de l'effluent butadiène brut par mise en contact avec une solution aqueuse basique ou une solution de bisulfite ou une solution aqueuse d'un composé de la famille des hydrazine, ne peut être facilement régénérée. Par conséquent, une quantité importante d'acétaldéhyde serait ainsi perdue, ce qui se traduirait par une baisse de rendement global du procédé.

La demanderesse a donc identifié un fonctionnement optimal du procédé en enchainant les étapes D2) et D2bis) de première et seconde purification du butadiène, permettant respectivement d'atteindre les spécifications, tout en maximisant le rendement global du procédé et en minimisant les coûts opératoires.

### Étape D3) ultérieure de purification du butadiène

Conformément à l'invention, une étape D3) ultérieure de purification du butadiène est alimentée au moins par ledit effluent butadiène pré-purifié issu de ladite étape D2), avantageusement traité dans l'étape D2bis) de seconde purification, et produit au moins un effluent butadiène purifié.

Cette étape D3) permet de purifier le butadiène produit dans les étapes réactionnelles à un très haut niveau de pureté (plus de 99,5% poids, préférentiellement plus de 99,8% poids, et très préférentiellement plus de 99,9% poids), tout en limitant les pertes de produit en séparant les impuretés n'ayant pas ou partiellement été retirées au cours de l'étape D1), D2) et avantageusement D2bis).

Dans un premier mode de l'invention, ladite étape D3) comprend au moins une section de séchage, une section de distillation cryogénique et une section de séparation butadiène/butènes par extraction liquide-liquide.

L'effluent butadiène pré-purifié issu de l'étape D2), avantageusement traité dans l'étape D2bis), alimente une section de séchage. Cette section a pour objectif d'atteindre les spécifications requises en eau dans le produit final (effluent butadiène purifié), et de permettre de pratiquer une séparation cryogénique sans risque de formation d'hydrates. En sortie de ladite section de séchage, on obtient un effluent butadiène sec. Par butadiène sec on entend moins de 10 ppm d'eau, de préférence moins de 5 ppm, de préférence moins de 1 ppm.

Ladite section de séchage comprend préférentiellement un séchage constitué d'une ou plusieurs capacités contenant un ou plusieurs adsorbants ayant une forte affinité pour l'eau. De manière non limitative, cet adsorbant peut être constitué de silice et/ou d'alumine. De manière non limitative, cet adsorbant peut être une zéolite telle qu'une zéolite 3A ou 4A. Lorsque l'adsorbant ou les adsorbants sont saturés en eau, on alimente ledit effluent butadiène pré-purifié vers une autre capacité contenant l'adsorbant ou les adsorbants frais ou régénérés.

La régénération de l'adsorbant peut être réalisée soit en modifiant le pression partielle d'eau au sein de la capacité, soit en modifiant la température au sein de la capacité, soit en modifiant la pression partielle d'eau et la température au sein de la capacité. Dans ce dernier mode de réalisation, la régénération du ou des adsorbants saturé en eau est faite en chauffant la capacité, tout en l'alimentant avec un flux ne contenant pas, ou très peu, d'eau. Par pas ou très peu d'eau, on entend moins de 500 ppm, préférentiellement moins de 350 ppm, de manière préférée moins de 10 ppm, de préférence moins de 5 ppm, de manière très préférée moins de 1 ppm. Ce flux ne contenant pas ou très peu d'eau peut être de façon non limitative un flux d'azote, un flux d'air, un flux d'hydrocarbure, ou un flux d'hydrogène. Dans un mode préféré de l'invention, on utilise une fraction de l'effluent hydrogène purifié issu de l'étape C1).

Ledit flux ne contentant pas ou très peu d'eau est chauffée à une température suffisante pour régénérer l'adsorbant ou les adsorbants avant d'être alimentée dans la capacité contenant l'adsorbant ou les adsorbants à régénérer, préférentiellement aux environs de 250°C.

Selon ce premier mode de réalisation, ledit effluent butadiène sec alimente ensuite une section de distillation cryogénique mettant en oeuvre une colonne à distiller. Les produits légers sortent en tête de la section de distillation cryogénique entre -25°C et -35°C. Le fond de la colonne est à une température comprise entre 20 et 50°C, préférentiellement entre 25 et 45°C, de manière très préférée entre 30 et 40°C, la pression en tête de colonne est comprise entre 0,3 et 0,4 MPa, préférentiellement 0,35 MPa. L'intérêt de la colonne est de présenter une très grande efficacité de séparation des derniers incondensables et ceci sans perte de butadiène (moins de 0.05 %). On évite ainsi un recyclage important vers l'étape D1) et une perte de butadiène.

Toujours selon ce premier mode de réalisation, le produit de fond de ladite section de distillation cryogénique, appelé effluent butadiène étêté, comprend pour principale impureté des butènes. Ledit effluent butadiène étêté alimente une section de séparation butadiène/butènes par extraction liquide-liquide, tel que décrite dans le brevet FR 2,036,057.

Ladite section de séparation butadiène/butènes est une section d'extraction liquide-liquide dans laquelle ledit effluent butadiène étêté alimente en une zone intermédiaire une première colonne d'extraction liquide-liquide, dans laquelle un flux de solvant polaire, préférentiellement du DMSO, est alimenté en tête. En fond, un solvant hydrocarbure saturé, préférentiellement du pentane ou du cyclohexane, est alimenté. Les débits ainsi que le ratio des débits de solvant polaire sur solvant hydrocarbure sont réglés de tel sorte que l'essentiel des butènes vont être entraînés par le solvant hydrocarbure et l'essentiel du butadiène être entraîné par le solvant polaire.

Le mélange butènes/hydrocarbure obtenu en tête de la première colonne d'extraction est ensuite traité dans une première colonne à distiller afin d'obtenir en tête l'effluent butènes, et en fond le solvant hydrocarbure qui peut être recyclé.

Le mélange butadiène/solvant polaire alimente ensuite la tête d'une deuxième colonne d'extraction liquide/liquide dans laquelle le butadiène est extrait du solvant polaire par mise en contact directe avec une quantité de solvant hydrocarbure plus importante que dans la première colonne d'extraction liquide-liquide, qui est introduite en fond de ladite deuxième colonne d'extraction liquide-liquide.

Le mélange butadiène/hydrocarbure obtenu en tête de la deuxième colonne d'extraction liquide-liquide est ensuite traité dans une colonne à distiller afin d'obtenir en tête l'effluent butadiène purifié, et en fond le solvant hydrocarbure qui peut être recyclé.

De manière préférée, les colonnes d'extraction liquide-liquide de ladite section de séparation butadiène/butènes sont opérées à une pression comprise entre 0,1 et 1 MPa, et une température comprise entre 20 et 60°C.

Dans un autre mode de l'invention, ladite étape D3) comprend au moins une distillation et une distillation extractive. L'étape de distillation peut être réalisée en amont ou en aval de l'étape de distillation extractive. La distillation extractive peut être réalisée de manière non limitative avec un solvant comme la N-methyl-pyrrolidone, le dimethyl-formamide ou l'acétonitrile.

Les différentes étapes de traitement et de purification du butadiène D1), D2), D2bis) et D3) peuvent bien évidemment également co-traiter tout flux comprenant du butadiène éventuellement produits par d'autres procédés situés à proximité du procédé selon l'invention.

### Étape E1) de traitement des effluents

Conformément à l'invention, l'étape E1) de traitement des effluents est alimentée au moins par le raffinat eau / éthanol / acétaldéhyde issu de l'étape E2) et produit au moins un effluent riche en éthanol, un effluent riche en acétaldéhyde et un effluent riche en eau. Si l'effluent eau usée issu de l'étape D2), ou l'effluent eau alcoolisée issu de l'étape F) ou l'effluent eau usée issu de l'étape C2) n'ont pas subi l'étape E2) d'élimination des impuretés et des huiles brunes, ils peuvent alimenter directement l'étape E1) de traitement des effluents. La section E1) est avantageusement également alimentée par une fraction de la charge éthanol.

De préférence et à la différence de l'art antérieur, aucun soutirage avec perte d'éthanol ou acétaldéhyde n'est effectué.

De préférence, ladite étape E1) comprend au moins deux sections de distillation. Une section de distillation dite de l'eau et de l'éthanol, et une section de distillation dite de l'acétaldéhyde.

Ledit effluent eau/éthanol/acétaldéhyde issu de l'étape E2) et éventuellement l'effluent eau usée issu de l'étape D2) alimentent ladite section de distillation de l'acétaldéhyde, dans laquelle l'acétaldéhyde est séparé de manière à former un effluent riche en acétaldéhyde, le résidu de ladite section de distillation de l'acétaldéhyde alimentant une section de distillation de l'eau et de l'éthanol permettant de séparer en tête un effluent riche en éthanol et en fond un effluent riche en eau. L'effluent eau alcoolisée issu de l'étape F) et l'effluent eau usée issu de l'étape C2) ne contenant pas d'acétaldéhyde, ils peuvent alimenter directement ladite section de distillation de l'eau et de l'éthanol.

L'effluent riche en éthanol issu de l'étape E1) est constitué majoritairement d'éthanol. Par majoritairement, on entend plus de 80%pds, de préférence plus de 84 % poids. De manière non limitative, l'effluent riche en éthanol issu de l'étape E1) peut contenir des impuretés comme de l'eau, de l'acétate d'éthyle, du butanol et de l'hexanol.

Les impuretés autre que l'eau représentent moins de 10%, de façon privilégiée moins de 5%, de façon encore préférentielle moins de 2% poids du flux.

L'effluent riche en acétaldéhyde issu de l'étape E1) est constitué majoritairement d'acétaldéhyde et d'éthanol. Par majoritairement, on entend plus de 80% pds, de préférence plus de 85 % poids. De manière non limitative, l'effluent riche en acétaldéhyde issu de l'étape E1) peut contenir des impuretés comme de l'eau, de l'acétate d'éthyle, de l'acétone. Les impuretés autre que l'eau représentent moins de 10%, de façon préférentielles moins de 5% poids du flux.

Lesdits effluents riche en acétaldéhyde, riche en éthanol et riche en eau sont ensuite recyclés dans le reste du procédé selon l'invention. La fraction dudit effluent riche en éthanol alimentant l'étape A) est de préférence au moins de 0,7, de préférence au moins 0,75, de manière très préférée au moins 0,8. La fraction dudit effluent riche en eau alimentant ladite étape F) est avantageusement compris entre 0 et 0,3, très avantageusement entre 0 et 0,1, plus avantageusement compris entre 0 et 0,01. La fraction dudit effluent riche en eau alimentant ladite étape E2) d'élimination des impuretés et des huiles brunes est avantageusement comprise entre 0 et 1, préférentiellement entre 0,3 et 0,6, et avantageusement entre 0,4 et 0,5.

Dans un autre mode de réalisation de l'invention, lesdits effluents riche en acétaldéhyde, riche en éthanol et riche en eau subissent un étape de purification avant d'être recyclés dans le reste du procédé. Par purification, on entend mettre en contact lesdits effluents avec des adsorbants comme par exemple du charbon actif, de la silice, de l'alumine ou encore une résine polymérique fonctionnalisée. Par exemple, un charbon actif permet d'éliminer les traces de butanol et d'hexanol comprises dans le flux riche en éthanol. Par exemple, une résine basique permet d'éliminer l'acide acétique présent dans l'effluent riche en eau. Quand les adsorbants sont saturés, et ne permettent pas de garantir la pureté des effluents riche en acétaldéhyde, riche en éthanol et riche en eau, ils sont soit éliminés, soit régénérés pour être réutilisés.

### Étape E2) d'élimination des impuretés liquides et des huiles brunes

Conformément à l'invention, une étape E2) d'élimination des impuretés et des huiles brunes est alimentée au moins par l'effluent éthanol/acétaldéhyde/eau issu de l'étape D1) et par une fraction de l'effluent eau issu de l'étape E1) et produit au moins un raffinat éthanol / acétaldéhyde / eau, un effluent huiles brunes légères et un effluent huiles brunes lourdes.

De préférence, ladite étape E2) comprend au moins une section de lavage/contre-lavage, une section de distillation des huiles brunes légères, et une section de distillation des huiles brunes lourdes.

Ladite section de lavage/contre-lavage préférentielle est alimentée en un point intermédiaire par ledit effluent éthanol/acétaldéhyde/eau issu de l'étape D1), avantageusement en mélange avec l'effluent eau usée issu de l'étape D2), l'effluent eau alcoolisée issu de l'étape F) et l'effluent eau usée issu de l'étape C2), si cette dernière étape est mise en oeuvre, et de manière préférée en mélange avec une fraction de l'effluent eau usée issu de l'étape D2). Ces effluents étant plus riches en eau que l'effluent éthanol/acétaldéhyde/eau issu de l'étape D1), leur introduction en mélange permet de limiter les pertes en hydrocarbure dans le raffinat.

Ladite section de lavage/contre-lavage préférentielle est alimentée en fond par un effluent hydrocarbures et en tête par une fraction de l'effluent eau issue de l'étape E1), qui ne comprend pas d'éthanol et d'acétaldéhyde. L'effluent hydrocarbures et la fraction de l'effluent eau issu de l'étape E1) sont alimentés à une température de préférence comprise entre 10 et 70°C, préférentiellement entre 45 et 55°C. Ladite section de lavage/contre-lavage produit en tête un extrait hydrocarbures de lavages chargée d'une fraction des impuretés et des huiles brunes, et en fond ledit raffinat éthanol / acétaldéhyde / eau.

Ladite section de lavage/contre-lavage est de préférence opérée à une pression comprise entre 0,1 et 0,5 MPa, préférentiellement entre 0,2 et 0,4 MPa. De préférence, l'ajout d'eau pour réaliser le contre-lavage est tel que la teneur en eau dans le raffinat eau / éthanol / acétaldéhyde est supérieure à 30% poids, de manière préférée supérieur à 40% poids.

Dans un mode de réalisation, le contact entre les deux phases liquides dans ladite section de lavage/contre-lavage est réalisé au sein d'un extracteur liquide-liquide. Différents modes de contact peuvent être envisagés. On peut citer de manière non limitative, une colonne garnie, une colonne puisée, ou bien une colonne compartimentée agitée. Dans un autre mode de réalisation, le contact entre les deux phases liquides dans ladite section de lavage/contre-lavage est réalisé au sein d'un contacteur membranaire, ou une cascade de contacteurs membranaires. Ce mode de contact est particulièrement bien adapté au système mis en oeuvre. En effet, les mélanges eau-éthanol-hydrocarbure sont connus pour former des émulsions stables, qui peuvent être problématique dans un extracteur liquide-liquide. Le contacteur membranaire permet de générer une aire de contact importante, favorisant le transfert des impuretés et des huiles vers la phase hydrocarbure, sans générer d'émulsion.

Ledit extrait hydrocarbures de lavages alimente ladite section de distillation des huiles brunes légères, laquelle produit en tant que distillat ledit effluent huiles brunes légères, et un résidu hydrocarbures comprenant la fraction lourde des huiles brunes.

Ledit effluent huiles brunes légères est composé d'impuretés produites par l'étape réactionnelle B), principalement du diéthyléther, d'acétate d'éthyle et du crotonaldéhyde, mais aussi la fraction légère des huiles brunes, composée d'impuretés en quantité plus faible, parmi lesquelles du pentène, de l'isoprène, du butanal, du vinyl ethyl ether. Cet effluent peut être brulé pour fournir une partie de la chaleur nécessaire au circuit d'huile chaude ou aux chaudières à vapeur du procédé, ou distillé pour récupérer un effluent diéthyléther et/ou un effluent acétate d'éthyle/crotonaldéhyde, qui pourra être soit valorisée, soit recyclée dans la section réactionnelle de l'étape B) pour être retransformé.

Ledit résidu hydrocarbures contient essentiellement les hydrocarbures servant au lavage, mais également la fraction la plus lourde des huiles brunes. Pour éviter l'accumulation des huiles brunes par le recyclage de l'effluent hydrocarbures vers l'extracteur liquide-liquide, une fraction dudit résidu hydrocarbures est traitée dans ladite section de distillation des huiles lourdes, consistant en une colonne à distiller, laquelle produit un distillat hydrocarbures composé pour l'essentiel d'hydrocarbures avec encore quelques traces d'huiles brunes et, en tant que résidu, ledit effluent huiles brunes lourdes comprenant plus de 80%, préférentiellement plus de 85% d'hydrocarbures ainsi que les huiles brunes les plus lourdes. La fraction dudit effluent hydrocarbures envoyée vers ladite section de distillation des huiles est comprise entre 5 et 30% du débit total dudit résidu hydrocarbures, et préférentiellement entre 10 et 20%. Le distillat hydrocarbures est mélangé à la fraction du résidu hydrocarbures qui n'a pas été traité dans ladite section de distillation des huiles lourdes afin de former l'effluent hydrocarbures alimentant ladite section de lavage/contre-lavage.

Cet effluent, qui représente de préférence entre 0,1 et 20% de la charge de ladite section de distillation des huiles lourdes, préférentiellement entre 0,3 et 5%, peut être brûlé pour fournir une partie de la chaleur nécessaire au circuit d'huile chaude ou aux chaudières à vapeur du procédé. Un appoint d'hydrocarbures équivalent aux pertes en fond de ladite section de distillation des huiles lourdes est nécessaire pour maintenir le débit de lavage constant. Cette colonne est réglée de manière à maintenir constante la concentration en huiles brunes dans la boucle de recyclage des hydrocarbures (boucle effluent hydrocarbures /effluent hydrocarbures de lavage).

Les effluents huiles brunes légères et lourdes sont éliminés du procédé.

L'effluent éthanol/acétaldéhyde/eau issu de l'étape D1) comprend principalement de l'éthanol, de l'acétaldéhyde, de l'eau mais aussi des impuretés telles que le diethyléther, l'acétate d'éthyle et les huiles brunes telles que définies précédemment. Ces impuretés peuvent s'accumuler si elles sont renvoyées vers les étapes réactionnelles A) et B) au sein de la coupe de distillation riche en acétaldéhyde et/ou de la coupe de distillation riche en l'éthanol et qu'elles ne sont que partiellement converties dans les sections réactionnelles des étapes A) et B). L'étape E2) permet de récupérer une partie de ces impuretés avant l'étape E1) de traitements des effluents, ce qui permet d'éviter la démixtion des huiles brunes au sein des colonnes à distiller, de simplifier le schéma de distillation, et d'obtenir à l'issue de l'étape E1) un effluent éthanol, un effluent acétaldéhyde et un effluent eau de plus grande pureté par rapport à l'art antérieur.

Le lavage de l'effluent éthanol/acétaldéhyde/eau issu de l'étape D1) avec un effluent hydrocarbures entraîne certaines impuretés, tandis que le contre-lavage du flux d'hydrocarbures entraine une partie des impuretés et des huiles brunes avec une fraction de l'effluent eau issu de l'étape E1), de manière à limiter toute perte en acétaldéhyde et en éthanol.

De manière surprenante, la demanderesse a découvert qu'il était possible d'obtenir une séparation de phase liquide-liquide en ajoutant certains hydrocarbures au résidu éthanol/acétaldéhyde issu de l'étape D1). Ce résultat est surprenant car le résidu éthanol/acétaldéhyde issu de l'étape est très riche en éthanol et acétaldéhyde qui sont miscibles en toute proportion avec les hydrocarbures. Par une sélection adéquate de l'hydrocarbure, la demanderesse a découvert qu'il était possible d'obtenir une séparation de phase liquide-liquide, et donc de réaliser une extraction liquide-liquide pour éliminer une partie des impuretés contenues dans l'effluent éthanol/acétaldéhyde/eau issu de l'étape D1). Ledit effluent hydrocarbures peut contenir des hydrocarbures saturés et/ou insaturés et /ou aromatiques, de préférence des hydrocarbures saturés. Ledit effluent hydrocarbures est avantageusement constitué d'un mélange d'hydrocarbures ayant entre 6 et 40 atomes de carbone, de préférence entre 10 et 20 atomes de carbone. De manière non limitative, ledit effluent hydrocarbures peut être une coupe gazole ou kérosène désulfurée ou bien encore une coupe d'hydrocarbures produite par une unité de type Fischer-Tropsh.

L'ajout d'eau au sein de la section de lavage/contre-lavage permet un meilleur fonctionnement du procédé d'élimination des impuretés et des huiles brunes selon l'invention.

Le procédé selon l'invention évite ainsi la purge régulière d'éthanol afin d'éviter l'accumulation des huiles brunes, ce qui permet d'améliorer les performances globales du procédé.

### Étape F) de lavage à l'eau des sous-produits gazeux

Conformément à l'invention, une étape F) de lavage à l'eau est alimentée par l'effluent sous-produits gazeux issu de l'étape D1), ainsi que par une fraction de l'effluent riche en eau issu de ladite étape E1) et produit au moins un effluent eau alcoolisée.

L'objectif de ladite étape F) est de récupérer la petite fraction d'éthanol entraînée dans ledit effluent sous-produits gazeux issu de l'étape D1) afin d'améliorer le rendement global du procédé.

La quantité d'eau issue de ladite étape E1) nécessaire dans ladite étape F) selon l'invention est très faible, contrairement à celle nécessaire dans l'art antérieur, car l'effluent vapeur issu de l'étape B) a été lavé dans l'étape D1) avec un flux éthanol contenant peu ou pas d'acétaldéhyde. Il ne reste donc dans ce flux qu'une petite fraction d'éthanol, facilement récupérée avec une faible quantité d'eau en comparaison avec la quantité d'eau qui aurait été nécessaire s'il y avait des traces d'acétaldéhyde dans l'effluent sous-produits gazeux issu de l'étape D1).

L'eau chargée en éthanol après le lavage est soutirée de ladite étape F) et constitue l'effluent eau alcoolisée. Elle alimente de préférence l'étape E1), directement dans la section de distillation eau-éthanol sans alourdir la section de distillation de l'acétaldéhyde. Dans un autre mode de l'invention, elle alimente de préférence l'étape E2) d'élimination des impuretés et des huiles brunes.

Le procédé selon l'invention permet donc de minimiser le débit des effluents à traiter dans l'étape de traitement des effluents. Il permet par ailleurs de réduire au maximum les pertes en butadiène, en permettant de récupérer plus de 98%, préférentiellement plus de 99 % du butadiène produit à l'issue des étapes réactionnelles dans ledit effluent butadiène purifié.

### DESCRIPTION DES FIGURES

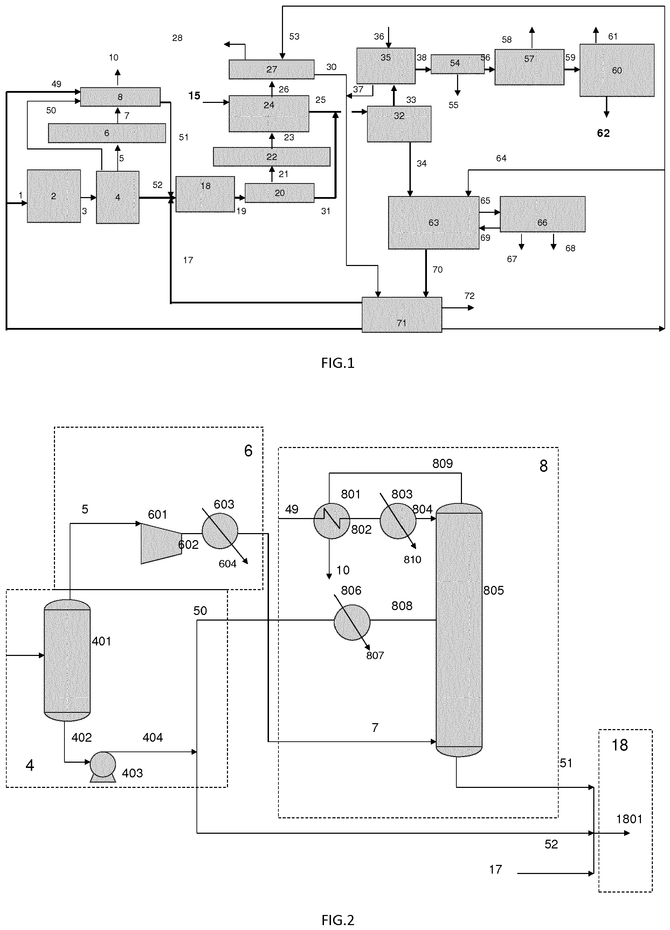

La **figure 1** représente de manière schématique et non limitative un arrangement du procédé selon l'invention.

Une fraction de l'effluent éthanol issu de la section de distillation 71 est envoyé par le conduit 1 vers la section réactionnelle 2, ou une partie de l'éthanol est converti principalement en acétaldéhyde et en hydrogène. L'effluent de cette section réactionnelle est envoyé par le conduit 3 vers la section de séparation 4.

La section de séparation 4 permet de séparer un effluent hydrogène 5, comprimé dans la section 6, et un effluent éthanol/acétaldéhyde, qui est séparé en deux fractions, l'une alimentant par le conduit 52 la section réactionnelle 18, l'autre envoyée vers la section de lavage 8 par le conduit 50 afin de laver l'effluent hydrogène comprimé 7. L'effluent hydrogène comprimé est également lavé avec une fraction de l'effluent éthanol 49 issu de la section de distillation 71 et évacué comme effluent hydrogène purifié par le conduit 10. Cet ensemble est décrit plus particulièrement **figure 2****.**

La section réactionnelle 18 est alimentée par l'effluent acétaldéhyde issu de la section de distillation 71 par le conduit 17, par l'effluent éthanol/acétaldéhyde ayant servi au lavage de l'hydrogène venant par le conduit 51, et par l'effluent éthanol/acétaldéhyde alimenté par le conduit 52 depuis la section de séparation 4. L'effluent de la section réactionnelle 18 est envoyé vers la section de séparation 20 par le conduit 19 pour être séparé en un effluent gazeux 21 et un effluent liquide 31.

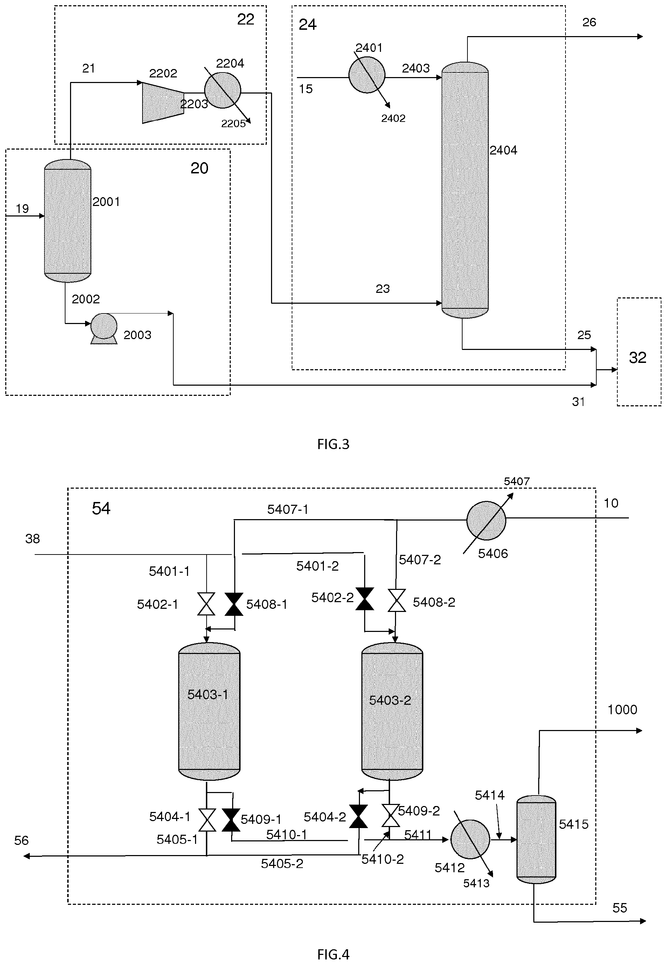

L'effluent gazeux 21 est comprimé dans la section 22. Il alimente via le conduit 23 une section de lavage 24 dans laquelle il est lavé par mise en contact avec la charge éthanol 15. Cet ensemble est plus particulièrement décrit **figure 3****.** L'effluent gazeux comprimé et lavé alimente par le conduit 26 une section de lavage à l'eau 27 dans laquelle il est lavé avec une fraction de l'effluent eau 53 issu de la section de distillation 71. L'eau chargée en éthanol après le lavage est renvoyée par le conduit 30 vers la section de distillation 71, directement dans la colonne de séparation eau-éthanol sans alourdir la colonne acétaldéhyde. L'effluent vapeur lavé dans la section 27 est soutiré par le conduit 28.

L'effluent liquide issu du séparateur 20, envoyé par le conduit 31 est mélangée au liquide de fond de lavage 24 arrivant par le conduit 25. Le mélange est envoyé à la distillation 32, qui va séparer en tête une coupe butadiène et en fond un mélange comprenant de eau, de l'éthanol, de l'acétaldéhyde et des impuretés. La coupe butadiène est envoyée par le conduit 33 vers un lavage à l'eau 35 destiné à éliminer les impuretés polaires et spécialement l'acétaldéhyde. L'eau de lavage, qui est de l'eau propre, est introduite par le conduit 36. L'eau chargée en acétaldéhyde est renvoyée par le conduit 37 vers la section de distillation 71.

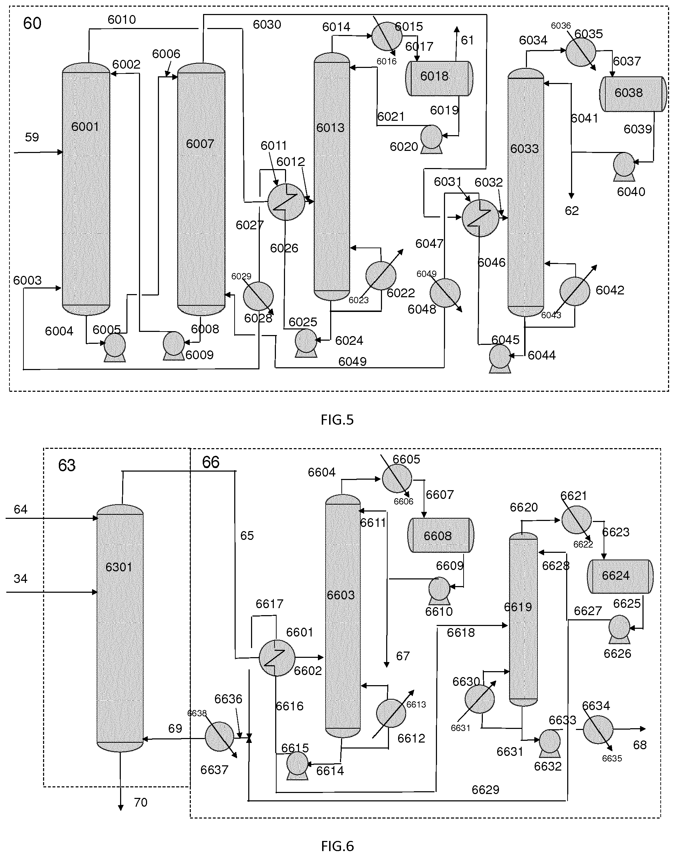

L'effluent butadiène pré-purifié est envoyé par le conduit 38 vers une section de séchage 54 afin d'éliminer toute trace d'eau. L'effluent butadiène sec 56 alimente une distillation cryogénique 57 et l'eau est évacuée par le conduit 55. Cet ensemble est décrit plus en détail **figure 4****.** Les produits légers sortent en tête de la section de distillation cryogénique à -35°C par la conduite 58, avec une perte très faible en butadiène. La coupe butadiène étêtée sort par le conduit 59 et arrive dans une section extraction liquide-liquide 60. Le fonctionnement de cette extraction est décrit plus en détail **figure 5****.**

L'effluent butadiène purifié sort de cette extraction par la conduite 62, à une pureté satisfaisante pour les spécifications actuelles (plus de 99.5%), les impuretés résiduelles étant principalement des butènes. Les butènes séparés dans cette section (comprenant une faible quantité de butadiène) sortent de l'unité par le conduit 61.

Le résidu éthanol/acétaldéhyde, produit de fond de la distillation 32, est envoyé par le conduit 34 vers la section 63 de lavage/contre-lavage aux hydrocarbures lourds, amenés par le conduit 69 et à l'eau recyclée amenée par le conduit 64. Les hydrocarbures lourds de lavage, chargés en impuretés sortent par le conduit 65 et alimentent la section de régénération 66, dont on va sortir les hydrocarbures lourds 69 retournant au lavage, une fraction légère 67, contenant en particulier du di-éthyle éther et de l'éthyle acétate, plus quelques huiles brunes légères. On sort également une coupe lourde 68, contenant des huiles brunes lourdes et une petite partie des hydrocarbures de lavage. Le fonctionnement des sections 63 et 66 est décrit plus en détail **figure 6****.**

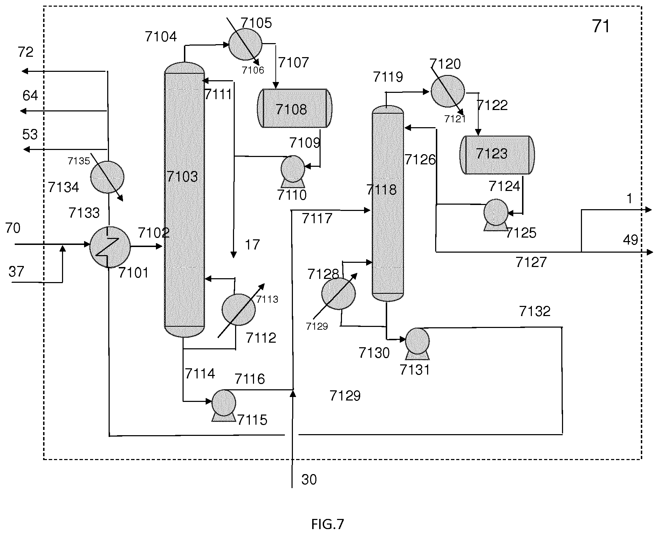

Le liquide de fond de la section de lavage/contre-lavage 63, contenant à la fois le résidu éthanol/acétaldéhyde 34 débarrassé de ses impuretés ayant de fortes affinités avec les hydrocarbures lourds 69 et l'eau de lavage 64, est envoyé par le conduit 70 vers la section de distillation 71. Cette section permet de séparer une fraction acétaldéhyde renvoyée par le conduit 17 vers la section réactionnelle 18, une fraction éthanol renvoyée en partie vers la section réactionnelle 2 par le conduit 1 et en partie vers le lavage 8 par le conduit 49, et une fraction eau, contenant un peu d'acide acétique, partiellement recyclée vers le lavage 27 par le conduit 53 et le lavage 63 par le conduit 64, le reste de l'eau étant purgée hors de l'unité par le conduit 72. Le fonctionnement de cette section est décrit en détails **figure 7****.**

La **figure 2** représente de manière schématique et non limitative la séparation de l'effluent de la section réactionnelle 2 et l'étape de traitement de l'hydrogène.

L'effluent de la section réactionnelle 2 alimente par le conduit 3 une capacité 401 permettant de séparer un effluent hydrogène 5 et une phase liquide 402. L'effluent hydrogène 5 est comprimée par le compresseur 601. L'effluent hydrogène comprimé est ensuite envoyé par le conduit 602 vers un échangeur de chaleur 603 qui va refroidir le gaz à l'aide d'une utilité froide 604. L'effluent hydrogène comprimé sort de l'échangeur de chaleur 603 par le conduit 7 et entre dans le fond de la colonne de lavage adiabatique 805 et va être lavé par deux liquides réfrigérés à basse température.

La phase liquide 402 est pompée par la pompe 403 à une pression plus élevée pour former l'effluent éthanol/acétaldéhyde 404. La fraction de l'effluent éthanol/acétaldéhyde 50 est refroidie par l'échangeur de chaleur 806 à l'aide d'un produit réfrigérant 807 qui peut être, par exemple, du propane. Le liquide sort de 806 par le conduit 808, avant d'entrer dans la colonne 805 à un niveau intermédiaire entre la tête et le fond de colonne.

La fraction de l'effluent éthanol issue de la section de distillation 71 est amenée par le conduit 49 à un premier échangeur de chaleur 801, permettant de refroidir l'éthanol de 49 et de réchauffer l'hydrogène sortant de la colonne 805 par le conduit 809. En sortie de cet échangeur, l'effluent hydrogène purifié, réchauffé, est sorti du procédé par le conduit 10, et l'éthanol refroidi est envoyé par le conduit 802 vers l'échangeur 803 pour le refroidir davantage, à l'aide d'un produit réfrigérant 810, qui peut être, par exemple, du propane. L'éthanol est envoyé par le conduit 804 dans la colonne 805. L'effluent liquide de fond de colonne 805 est envoyé par le conduit 51 vers la section réactionnelle 18, en mélange avec la fraction 52 de l'effluent éthanol/acétaldéhyde 404.

La **figure 3** représente de manière schématique et non limitative la séparation de l'effluent de la section réactionnelle 18 et une partie de l'étape de traitement de l'effluent butadiène.

L'effluent 19 de la section réactionnelle 18 alimente un séparateur 2001 dans lequel un effluent butadiène 21 et une phase liquide 2002 sont séparées. L'effluent butadiène 21 est comprimée dans un compresseur 2202, l'effluent vapeur comprimé 2203 étant ensuite refroidi dans un échangeur de chaleur 2204 par une utilité froide 2205.

L'effluent vapeur comprimé et refroidi 23 alimente la colonne de lavage adiabatique 2404, où il va être lavé par la charge éthanol 15, préalablement refroidie dans l'échangeur de chaleur 2401 par un produit réfrigérant arrivant par le conduit 2402. La charge éthanol préalablement refroidie arrive par le conduit 2403 dans la colonne 2404. On récupère en tête de colonne un effluent vapeur comprimé et lavé 26 et en fond le liquide de fond de lavage 25.

Le liquide de fond de lavage 25 est mélangé avec la phase liquide 2002 qui a été au préalable pompée par la pompe 2003 et arrivant par le conduit 31. Le mélange de 25 et 31 contient la totalité du butadiène produit, et est envoyé vers la section 32.

La **figure 4** représente de manière schématique et non limitative le séchage du butadiène sur tamis.

L'effluent butadiène pré-purifié arrive par le conduit 38 pour être séché, il passe par la vanne ouverte 5402-1 ou 2 puis par le tamis de séchage contenu dans la capacité 5403-1 ou 2. Le butadiène sec sort de la capacité 5403-1 ou 2 à travers la vanne ouverte 5404-1 ou 2, puis le conduit 5405-1 ou 2 et est enfin envoyé à la distillation cryogénique par le conduit 56.

Le flux d'hydrogène arrive par le conduit 10, est chauffé dans l'échangeur de chaleur 5406 par échange avec un moyen de chauffe 5407, qui peut être par exemple de l'huile chaude.

À la sortie de l'échangeur de chaleur 5406, le flux d'hydrogène chaud est envoyé par le conduit 5407-2 ou 5407-1 vers la vanne ouverte 5408-2 ou 1, et de là vers la capacité en régénération 5403-2 ou 1. A la sortie de la capacité 5403-2 ou 1, le gaz chaud chargé en eau passe par la vanne ouverte 5409-2 ou 1, puis par le conduit 5410-2 ou 1, le conduit 5411 , puis l'échangeur de chaleur 5412, ou il est refroidi et l'eau condensée à l'aide de l'eau de refroidissement arrivant par le conduit 5413. Le gaz peut être refroidi aussi bien par un aéroréfrigérant. Le gaz refroidi en sortie de 5412 est envoyé à travers le conduit 5414 vers le séparateur 5415, ou l'eau condensée est séparée, et envoyée hors de l'unité par le conduit 55. L'eau condensée contient une partie de l'éthanol contenue dans le flux 10, elle peut éventuellement être envoyée vers la section 71 pour récupérer l'éthanol.

La **figure 5** représente de manière schématique et non limitative la purification du butadiène à l'aide d'un solvant polaire, par exemple du DMSO (diméthyle sulfoxyde).

L'effluent butadiène sec alimente par le conduit 59 une première colonne d'extraction 6001, dans laquelle un flux de solvant polaire, qui peut être par exemple du DMSO, arrive en tête par le conduit 6002. En fond, un solvant hydrocarbure, tel qu'un pentane ou du cyclohexane est alimenté par le conduit 6003.

En fond de la colonne 6001, le solvant polaire et le butadiène dissous sortent par le conduit 6004, sont pompés par la pompe 6005, et sont envoyés en tête de la colonne 6007 par le conduit 6006. Une plus grande quantité de solvant hydrocarbure est injecté en fond de la colonne 6007 par le conduit 6049 afin de sortir le butadiène du solvant polaire. En fond de 6007, le solvant polaire débarrassé du butadiène sort par le conduit 6008 et est pompé par la pompe 6009 puis renvoyé vers la colonne 6001 par le conduit 6002.

En tête de colonne, le butadiène dissous dans les hydrocarbures est envoyé par le conduit 6030 vers l'échangeur de chaleur 6031, ou il est chauffé par échange indirect avec le fond de la colonne 6033. En sortie de l'échangeur de chaleur 6031, le mélange butadiène-solvant est alimenté dans la colonne 6033 par le conduit 6032.

Cette colonne 6033 est pourvue d'un dispositif 6042 permettant de chauffer et rebouillir le fond de la colonne à l'aide d'un moyen de chauffe 6043, qui peut être par exemple de la vapeur d'eau à basse pression. En tête de colonne, la vapeur de tête sortant par le conduit 6034 est refroidie et totalement condensée dans l'échangeur de chaleur 6035, utilisant une utilité froide 6036. En sortie de l'échangeur de chaleur 6035, le liquide condensé coule dans la capacité 6038 par le conduit 6037. Le liquide est ensuite envoyé par le conduit 6039 vers la pompe 6040. Une partie du liquide est envoyé comme reflux à la colonne 6033 par le conduit 6041, le reste, qui constitue l'effluent butadiène purifié est envoyé hors du procédé par le conduit 62.

Le fond de la colonne 6033, qui est du solvant hydrocarbure, est envoyé par le conduit 6044 à la pompe 6045. En sortie de pompe 6045, le solvant est envoyé par le conduit 6046 vers l'échangeur de chaleur 6031, où il est refroidi par échange indirect de chaleur avec la charge de la colonne 6033. En sortie de l'échangeur de chaleur 6031, le solvant est envoyé par le conduit 6047 à l'échangeur de chaleur 6048, pour finir le refroidissement à l'aide d'une utilité froide 6049. En sortie de l'échangeur 6048, le solvant est renvoyé à la colonne de lavage 6007 par le conduit 6049.

En tête de la colonne 6001 sort un mélange solvant hydrocarbure et butènes, avec une petite perte en butadiène. Ce mélange est envoyé à l'échangeur de chaleur 6011, où il est chauffé par échange indirect avec le fond de la colonne 6013. En sortie de l'échangeur de chaleur 6011, le mélange butène-solvant alimente la colonne 6013 par le conduit 6012. Cette colonne 6013 est pourvue d'un dispositif 6022 permettant de chauffer et rebouillir le fond de la colonne à l'aide d'un moyen de chauffe 6023, qui peut être par exemple de la vapeur d'eau à basse pression. En tête de colonne, la vapeur de tête sortant par le conduit 6014 est refroidie et totalement condensée dans l'échangeur de chaleur 6015, utilisant une utilité froide 6016. En sortie de l'échangeur de chaleur 6015, le mélange partiellement condensé est alimenté dans la capacité 6018 par le conduit 6017. Les phases gaz et liquide sont séparées dans cette capacité 6018. La phase liquide de la capacité est envoyée par le conduit 6019 vers la pompe 6020, puis envoyé comme reflux à la colonne 6013 par le conduit 6021. La phase vapeur, constituée essentiellement de butènes et d'un peu de butadiène est sortie du procédé par le conduit 61 pour servir, par exemple, de combustible.

Le fond de la colonne 6013, qui est du solvant hydrocarbure, est envoyé par le conduit 6024 à la pompe 6025. En sortie de pompe 6025, le solvant est envoyé par le conduit 6026 à l'échangeur de chaleur 6011, où il est refroidi par échange indirect de chaleur avec la charge de la colonne 6013. En sortie de l'échangeur de chaleur 6011, le solvant est envoyé par le conduit 6027 à l'échangeur de chaleur 6028, pour finir le refroidissement à l'aide d'une utilité froide 6029. En sortie de l'échangeur 6028, le solvant est renvoyé à la colonne de lavage 6001 par le conduit 6003.

La **figure 6** représente de manière schématique et non limitative l'extraction des impuretés peu polaires et des huiles brunes par lavage/contre-lavage.

Le résidu éthanol/acétaldéhyde 34 alimente la colonne de lavage 6301. Le solvant hydrocarbures lourds (qui peut être par exemple du coupe gasoil ou kérosène désulfuré, ou une coupe produite par une unité type Fischer-Tropsh) est alimenté en fond de colonne 6301 par le conduit 69, tandis qu'une fraction de l'effluent eau 64 alimente la colonne 6301 en tête de colonne.

L'effluent hydrocarbures lourds de lavage est soutiré en tête de la colonne 6301 par le conduit 65 et est envoyé dans la section 66 pour être régénéré. Il est préchauffé dans l'échangeur de chaleur 6601 par échange avec le fond de la colonne 6603. À la sortie de l'échangeur 6601, L'effluent hydrocarbures lourds de lavage préchauffé est envoyé par le conduit 6602 vers la colonne 6603.

Cette colonne 6603 est pourvue d'un dispositif 6612 permettant de chauffer et rebouillir le fond de la colonne à l'aide d'un moyen de chauffe 6613, qui peut être par exemple de l'huile chaude. En tête de colonne, la vapeur de tête sortant par le conduit 6604 est refroidie et totalement condensée dans l'échangeur de chaleur 6605, utilisant une utilité froide 6606. En sortie de l'échangeur de chaleur 6605, le liquide condensé coule dans la capacité 6608 par le conduit 6607. Le liquide est ensuite envoyé par le conduit 6609 vers la pompe 6610. Une partie du liquide est envoyé comme reflux à la colonne 6603 par le conduit 6611, le reste est envoyé hors de l'unité par le conduit 67.

Le fond de la colonne 6603 est envoyé par le conduit 6614 à la pompe 6615. Une fraction du liquide en sortie de pompe 6615 est envoyé par le conduit 6618 vers une autre colonne à distiller 6619. La fraction restante est envoyée par le conduit 6616 vers l'échangeur de chaleur 6601, qui va permettre de refroidir le liquide de fond 6616 par échange indirect avec la charge 65 de la colonne 6603. Le liquide de fond refroidi sort de l'échangeur 6601 par le conduit 6617 pour être envoyé à l'échangeur de chaleur 6637.

La colonne de purification 6619 est pourvue d'un dispositif 6630 permettant de chauffer et rebouillir le fond de la colonne à l'aide d'un moyen de chauffe 6631, qui peut être par exemple de l'huile chaude. En tête de colonne, la vapeur de tête sortant par le conduit 6620 est refroidie et totalement condensée dans l'échangeur de chaleur 6621, utilisant une utilité froide 6622. En sortie de l'échangeur de chaleur 6621, le liquide condensé coule dans la capacité 6624 par le conduit 6623. Le liquide est ensuite envoyé par le conduit 6625 vers la pompe 6626. Une partie du liquide est envoyé comme reflux à la colonne 6619 par le conduit 6628, le reste est envoyé par le conduit 6629 vers l'échangeur 6637 en mélange avec le fond de la colonne 6603 où il est refroidi à l'aide d'une utilité froide 6638. Les hydrocarbures en sortie de 6637 sont renvoyés par le conduit 69 vers la colonne de lavage 6301.

La tête de colonne 6619 est composée pour l'essentiel d'hydrocarbures lourds avec encore quelques traces d'"huile noire". Un appoint équivalent d'hydrocarbures lourds (non représenté) est nécessaire pour maintenir le débit de lavage constant.

Le liquide de fond de la colonne 6619 sort par le conduit 6631 et est pompé par la pompe 6632 puis envoyé par le conduit 6633 vers un échangeur de chaleur 6634, ou il est refroidi par un fluide 6635, pour être sorti du procédé par le conduit 68. Il pourra ensuite servir, par exemple, de combustible. Le fluide 6635 peut être de l'eau de refroidissement, il est possible aussi d'utiliser de l'air avec un aéroréfrigérant, ou d'utiliser pour ce fluide 6635 un flux de l'unité devant être réchauffé.

La **figure 7** présente un arrangement possible pour la section de distillation 71.

Le mélange éthanol/acétaldéhyde/eau/impuretés polaires issu du lavage 63 est amené par le conduit 70 vers la section de distillation 71. Ce flux est mélangé avec l'eau de lavage chargé en acétaldéhyde arrivant par le conduit 37 de la section de lavage 35. Le mélange de ces deux flux est chauffé par échange thermique indirect contre le flux 7133 dans l'échangeur de chaleur 7101. La sortie de cet échangeur est amenée à la colonne 7103 par le conduit 7102. La tête de la colonne sortant par le conduit 7104, est totalement condensée dans l'échangeur de chaleur 7105 par échange avec une utilité froide 7106. La sortie de l'échangeur 7105 est amenée par le conduit 7107 vers le ballon de reflux 7108. Le liquide de ce ballon sort par le conduit 7109 vers la pompe 7110, qui envoie un reflux vers la colonne 7103 par le conduit 7111 et un distillat (effluent riche en acétaldéhyde) par le conduit 17 vers la section réactionnelle 18. Ce distillat comporte principalement de l'acétaldéhyde, mais aussi de l'eau, de l'éthanol et d'autres impuretés légères (diéthyl éther, butanal, acétone, éthyl acétate, etc).

La colonne 7103 est rebouillie à l'aide de l'échangeur 7112 avec par exemple de la vapeur basse pression arrivant par le conduit 7113. Le produit de fond de la colonne 7103, contenant principalement de l'eau, de l'éthanol, un peu de butanol, de l'acide acétique et quelques autres impuretés sort par le conduit 7114, puis est envoyé à l'aide de la pompe 7115 par le conduit 7116 vers la colonne 7118. L'eau de lavage de la section de lavage 27, chargée en éthanol arrive par le conduit 30 et est mélangée avec le produit de fond de 7103 arrivant par le conduit 7116. Le mélange est envoyé par le conduit 7117 vers la colonne 7118.

La tête de la colonne 7118 sort par le conduit 7119, puis est totalement condensée par l'échangeur de chaleur 7120 à l'aide d'une utilité froide 7121. La sortie de l'échangeur 7120 est amenée par le conduit 7122 vers le ballon de reflux 7123. Le liquide de ce ballon sort par le conduit 7124 vers la pompe 7125, qui envoie un reflux vers la colonne 7118 par le conduit 7126 et un distillat par le conduit 7127, en partie vers la section réactionnelle 2 par le conduit 1, l'autre partie vers le lavage 8 par le conduit 49 (effluent riche en éthanol). L'ajustement du ratio entre ces deux destinations permet d'ajuster le ratio Ethanol/acétaldéhyde à l'entrée du réacteur de la section 18. Ce distillat comporte principalement de l'éthanol, mais également de l'eau, un peu de butanol, et quelques autres impuretés.

La colonne 7118 est rebouillie à l'aide de l'échangeur 7128 avec par exemple de la vapeur basse pression arrivant par le conduit 7129. Le produit de fond de la colonne 7118, (effluent riche en eau) contenant principalement de l'eau, et un peu d'acide acétique sort par le conduit 7130, est envoyé à l'aide de la pompe 7131 par le conduit 7132 vers l'échangeur de chaleur 7101 où il est refroidi par échange indirect avec la charge de la colonne 7103. Le produit sort de 7101 par le conduit 7133 et est refroidi dans l'échangeur 7134 à l'aide d'une utilité froide 7135. À la sortie de l'échangeur, une partie de l'eau est envoyé par le conduit 64 vers le lavage 63 et par le conduit 53 vers le lavage 27, le reste est purgé par le conduit 72 à l'extérieur de l'unité.

### EXEMPLES

Les exemples suivants sont basés sur des simulations procédés prenant en compte les recyclages des flux, et intégrant des données thermodynamiques calées sur des points expérimentaux (données d'équilibre liquide-vapeur binaires et coefficient de partage liquide-liquide). Dans chacun des exemples, le débit de charge est ajustée de manière à obtenir une production annuelle de 150 kt/an d'un butadiène ayant une pureté comprise entre 99,5 et 100% poids (en adéquation avec l'utilisation actuelle du produit), avec une durée de fonctionnement annuelle du procédé de 8000 h.

### Exemple 1 - Procédé de production de butadiène selon l'art antérieur

### 1.1 - Étape de traitement de l'hydrogène

La phase gaz en sortie du réacteur de conversion de l'éthanol en acétaldéhyde, qui représente un débit de 10 t/h, comprend de l'hydrogène coproduit par la réaction, de l'éthanol et de l'acétaldéhyde. Il est important de limiter au maximum les pertes en éthanol et acétaldéhyde afin de maximiser le rendement global du procédé. Le lavage de la phase gaz par de l'eau permet de récupérer 95,5% poids de l'acétaldéhyde et de l'éthanol contenus dans cette phase gaz.

Cet objectif de lavage est atteint en utilisant 50 t/h d'eau. Ces 50 t d'eau peuvent être uniquement de l'eau propre, de source externe au procédé, ou être en partie de l'eau recyclée provenant du process. La perte en éthanol dans l'effluent hydrogène purifié est de 31 kg/h.

L'utilisation d'eau recyclée permet de minimiser la consommation d'eau propre, mais, par effet de recyclage, va emmener de l'acide acétique dans l'effluent hydrogène purifié. Au maximum 40 t/h d'eau recyclée peut être utilisée. La consommation d'eau « propre » sera donc, au minimum, de 10 t/h. Ces 10 à 50 t/h d'eau représentent à elles seules entre 35 et 70 % du flux d'eau traité dans l'ensemble du schéma procédé de l'art antérieur.

Que l'eau de lavage provienne en partie d'eau recyclée ou totalement d'eau propre, le débit d'eau entrant dans la section de distillation eau/éthanol/acétaldéhyde s'élève à 142 t/h.

### 1.2 - Étape de première séparation du butadiène, alternative 1

L'effluent vapeur de l'étape de conversion en butadiène est comprimé, puis lavé par mise en contact avec l'effluent éthanol/acétaldéhyde issu du réacteur de conversion de l'éthanol en acétaldéhyde, préalablement refroidi à 35°C. Le lavage dudit effluent vapeur par ledit effluent éthanol/acétaldéhyde permet de récupérer la presque totalité du butadiène (99,988%) compris dans ledit effluent vapeur avant lavage. L'effluent vapeur en sortie de lavage forme l'effluent gaz lavé. Il comprend les sous-produits réactionnels (éthylène, éthane, etc...), mais également de l'éthanol et de l'acétaldéhyde.

Ledit effluent gaz lavé est ensuite lavé avec de l'eau recyclée pour récupérer l'acétaldéhyde et l'éthanol. Il est nécessaire d'employer 12,7 t/h d'eau pour récupérer l'ensemble de l'acétaldéhyde et de l'éthanol.

### 1.3 - Étape de première séparation du butadiène, alternative 2

Cet exemple se différencie de l'exemple précédent en ce que l'effluent éthanol/acétaldéhyde issu du réacteur de conversion de l'éthanol en acétaldéhyde est préalablement refroidi à 14°C avant d'être utilisé pour laver l'effluent vapeur de l'étape de conversion en butadiène préalablement comprimé.

La diminution de la température de l'effluent éthanol/acétaldéhyde permet de récupérer l'intégralité du butadiène compris dans ledit effluent vapeur et de réduire la quantité d'éthanol et d'acétaldéhyde entrainée dans l'effluent gaz lavé.

Cet effluent gaz lavé est ensuite lavé avec de l'eau recyclée pour récupérer l'acétaldéhyde et l'éthanol. Il est nécessaire d'employer 8,5 t/h d'eau pour récupérer l'ensemble de l'acétaldéhyde et de l'éthanol.

### Exemple 2 - Procédé de production de butadiène selon l'invention

### 2.1 - Étape de traitement de l'hydrogène

La phase gaz en sortie du réacteur de conversion de l'éthanol en acétaldéhyde est lavée, après compression, avec une fraction de l'effluent éthanol issu de l'étape de traitement des effluents. La phase gaz lavée forme l'effluent hydrogène purifié. La perte en éthanol dans l'effluent hydrogène purifié est de 32 kg/h, similaire à la perte de 31 kg/h d'acétaldéhyde dans le schéma de l'art antérieur.

Le lavage avec de l'éthanol recyclé en lieu et place d'eau permet de réduire de 50 t/h le débit d'eau en entrée de la section de distillation eau/éthanol/acétaldéhyde, qui est donc réduit à 91 t/h. Cela représente un gain de 35 % sur le débit d'eau circulant dans l'ensemble du schéma procédé, et en particulier dans les colonnes de la section de distillation. Ce gain se traduit par une baisse du niveau des investissements et de la consommation des utilités sur toute la section de récupération de l'éthanol et de l'acétaldéhyde.

### 2.2 - Étape de première séparation du butadiène, alternative 1

Dans cet exemple, l'effluent vapeur de l'étape de conversion en butadiène est comprimé, puis lavé par mise en contact avec la charge éthanol du procédé de conversion. L'effluent gaz en sortie de lavage forme l'effluent gaz lavé.

Ladite charge éthanol a la composition suivante : 93,3% poids en éthanol et 6,7 % d'eau, sans trace mesurable d'impuretés. Elle alimente la colonne de lavage à une température de 35°C. Ce lavage permet de récupérer 99,88% du butadiène compris dans ledit effluent vapeur. L'effluent gaz lavé est exempt d'acétaldéhyde, contrairement aux exemples selon l'art antérieur.

L'effluent gaz lavé est ensuite lavé avec de l'eau recyclée pour récupérer l'éthanol. Il est nécessaire d'employer 3,2 t/h d'eau pour récupérer l'ensemble de de l'éthanol, soit bien moins que dans les exemples selon l'art antérieur.

### 2.3 - Étape de première séparation du butadiène, alternative 2

Cet exemple se différencie du précédent en ce que la charge éthanol est préalablement refroidie à 14°C avant d'être utilisée pour le lavage de l'effluent vapeur de l'étape de conversion en butadiène préalablement comprimé. Ce lavage permet de récupérer la totalité du butadiène compris dans ledit effluent vapeur.

L'effluent gaz lavé, exempt d'acétaldéhyde, est ensuite lavé avec de l'eau recyclée pour récupérer l'éthanol. Il est nécessaire d'employer 0,4 t/h d'eau pour récupérer l'ensemble de de l'éthanol, soit plus de 20 fois moins que selon l'art antérieur.

La diminution du débit d'eau requis pour le lavage permet de réduire de 13% le débit d'eau en entrée de l'étape de traitement des effluents, réduisant par conséquent la taille des équipements de séparation et leur consommation énergétique.

Cette variante privilégiée de l'invention en utilisant la charge éthanol par rapport à l'emploi d'une fraction de l'effluent éthanol issu de l'étape de traitement des effluents permet de réduire le débit de flux lavant la coupe butadiène. Le débit en éthanol est alors réduit jusqu'à 16 %.

### 2.4 - Étape de première séparation du butadiène, alternative 3

Dans cet exemple, l'effluent vapeur de l'étape de conversion en butadiène est comprimé, puis lavé par mise en contact avec une fraction de l'effluent éthanol issu de l'étape de traitement des effluents. L'effluent gaz en sortie de lavage forme l'effluent gaz lavé.

Ledit effluent éthanol issu de l'étape de traitement des effluents a la composition suivante : 84% poids en éthanol et 16 % d'eau, sans trace mesurable d'impuretés. Il alimente la colonne de lavage à une température de 35°C. Ce lavage permet de récupérer la totalité du butadiène compris dans ledit effluent vapeur. L'effluent gaz lavé est exempt d'acétaldéhyde, contrairement aux exemples selon l'art antérieur.

L'effluent gaz lavé est ensuite lavé avec de l'eau recyclée pour récupérer l'éthanol. Il est nécessaire d'employer 2,8 t/h d'eau pour récupérer l'ensemble de de l'éthanol, soit bien moins que dans les exemples selon l'art antérieur.

### 2.5 - Étape de première séparation du butadiène, alternative 4

Cet exemple se différencie du précédent en ce que la fraction de l'effluent éthanol issu de l'étape de traitement des effluents est préalablement refroidie à 14°C avant d'être utilisée pour le lavage de l'effluent vapeur de l'étape de conversion en butadiène préalablement comprimé. Ce lavage permet de récupérer la totalité du butadiène compris dans ledit effluent vapeur.

L'effluent gaz lavé, exempt d'acétaldéhyde, est ensuite lavé avec de l'eau recyclée pour récupérer l'éthanol. Il est nécessaire d'employer 0,4 t/h d'eau pour récupérer l'ensemble de de l'éthanol, soit plus de 20 fois moins que selon l'art antérieur.

La diminution du débit d'eau requis pour le lavage permet de réduire de 13% le débit d'eau en entrée de l'étape de traitement des effluents, réduisant par conséquent la taille des équipements de séparation et leur consommation énergétique.

### 2.6 - Étape de séparation ultérieure du butadiène

L'effluent butadiène purifié issu de l'étape de première purification du butadiène alimente une section de séchage, par passage successif sur une alumine, puis sur une zéolite 4A, afin d'abattre la totalité de l'eau éventuellement présente dans ledit effluent butadiène purifié. L'effluent de la section de séchage forme l'effluent butadiène sec. Cet effluent butadiène sec alimente ensuite une colonne de distillation cryogénique, opérée avec une pression en tête de 0,35 Mpa, une température de fond de 34°C et une température de tête de -33°C.

Le résidu de distillation, appelé effluent butadiène étêté, alimente enfin une section d'extraction liquide-liquide mettant en oeuvre du DMSO et du cyclohexane.

Une première colonne de lavage est alimentée en tête par 260 t/h de DMSO et en fond par 52 t/h de cyclohexane. Cette première colonne comprend 20 étages théoriques de lavage. Le produit de fond de la première colonne est envoyé vers une deuxième colonne de lavage comprenant 10 étages théoriques.

Le produit de tête de cette seconde colonne de lavage est traité dans une colonne à distiller, qui permet de séparer le butadiène du cyclohexane, comprenant 24 étages théoriques et opérée avec un taux de reflux de 8.

Le produit de tête de la première colonne de lavage est traité dans une colonne à distiller, qui permet de séparer les butènes du cyclohexane, comprenant 26 étages théoriques et opérée avec un taux de reflux de 10.

99.3% du butadiène entrant dans la l'étape de seconde séparation du butadiène est récupéré en tant que produit, avec une pureté de 99.88% poids

On obtient en sortie de section d'extraction liquide-liquide un effluent butadiène purifié dont la teneur en butadiène est de 99,88% poids. Les pertes en butadiène sur l'ensemble des étapes de purification (calculées à partir du ratio du débit de butadiène pur compris dans l'effluent butadiène purifié sur le débit de butadiène pur compris dans l'effluent du réacteur de conversion en butadiène) est inférieur à 0,8 % poids.

L'agencement des étapes et des recyclages selon l'invention, en particulier en évitant l'accumulation d'impuretés, permet de recycler la quasi-totalité des composés n'ayant pas réagi. Ainsi, malgré une conversion par passe dans les réacteurs faible, et comparable à l'art antérieur, le rendement global en t_{butadiène produit} par t_{éthanol converti} est de 41%. La mise en oeuvre des recyclages selon l'invention permet d'améliorer le rendement global de plus de 20 point par rapport à une situation sans recyclage ainsi qu'une récupération et une valorisation de la totalité de l'acétaldéhyde présent dans l'effluent de la deuxième étape de conversion. Ainsi, plus de 99,9% de l'éthanol compris dans la charge du procédé est valorisée.

## Revendications

1. Procédé de production de butadiène à partir d'une charge éthanol comprenant au moins 80% poids d'éthanol comprenant au moins :
A) une étape de conversion de l'éthanol en acétaldéhyde comprenant au moins une section réactionnelle alimentée au moins par une fraction de l'effluent riche en éthanol issu de l'étape E1), opérée à une pression comprise entre 0,1 et 1,0 MPa et à une température comprise entre 200 et 500°C en présence d'un catalyseur, et une section de séparation permettant de séparer l'effluent de ladite section réactionnelle en au moins un effluent hydrogène sous forme gazeuse et un effluent éthanol/acétaldéhyde sous forme liquide ;
B) une étape de conversion en butadiène comprenant au moins une section réactionnelle alimentée au moins par une fraction dudit effluent éthanol/acétaldéhyde issu de l'étape A), par un effluent liquide riche en éthanol issu de l'étape C1), par une fraction de l'effluent riche en acétaldéhyde issu de l'étape E1), opérée en présence d'un catalyseur, à une température comprise entre 300 et 400°C, et à une pression comprise entre 0,1 et 1,0 MPa, les débits d'alimentation étant réglés de telle sorte que le rapport molaire éthanol/acétaldéhyde en entrée de ladite section réactionnelle est compris entre 1 et 5, et une section de séparation permettant de séparer l'effluent de ladite section réactionnelle en au moins un effluent gazeux et un effluent liquide ;
C1) une étape de traitement de l'hydrogène comprenant au moins une section de compression comprimant ledit effluent hydrogène issu de l'étape A) à une pression comprise entre 0,1 et 1,0 MPa, et une section de lavage gaz-liquide alimentée à une température comprise entre 15°C et -30°C par une fraction dudit effluent éthanol issu de l'étape E1) et par une fraction dudit effluent éthanol/acétaldéhyde issu de l'étape A), et alimentée à une température comprise entre 25 et 60°C par ledit effluent hydrogène comprimé, et produisant au moins un effluent liquide riche en éthanol et un effluent hydrogène purifié ;
D1) une étape d'extraction du butadiène comprenant au moins une section de compression comprimant ledit effluent gazeux issu de l'étape B) à une pression comprise entre 0,1 et 1,0 MPa, une section de lavage gaz-liquide comprenant une colonne de lavage alimentée en tête à une température comprise entre 20 et -20°C par un flux éthanol constitué de ladite charge éthanol du procédé et/ou d'une fraction de l'effluent éthanol issu de l'étape E1) et en fond par ledit effluent gazeux issue de l'étape B) et refroidi, et une section de distillation opérée à une pression comprise entre 0,1 et 1 MPa, alimentée au moins par l'effluent liquide issu de ladite étape B) et par l'effluent liquide de ladite section de lavage gaz-liquide, ladite étape D1) produisant au moins un effluent sous-produits gazeux, un effluent butadiène brut, et un effluent éthanol / acétaldéhyde / eau;
D2) une étape de première purification du butadiène comprenant au moins une section de lavage gaz-liquide alimentée en fond par l'effluent butadiène brut issu de D1) et en tête par un flux d'eau pouvant être un flux d'eau d'origine externe audit procédé de production de butadiène et/ou une fraction de l'effluent eau issu de l'étape E1), ladite section de lavage produisant en tête un effluent butadiène pré-purifié et en fond un effluent eau usée ;
D3) une étape ultérieure de purification du butadiène, alimentée au moins par ledit effluent butadiène pré-purifié issu de ladite étape D2), et produisant au moins un effluent butadiène purifié ;
E1) une étape traitement des effluents alimentée au moins par le raffinat eau / éthanol / acétaldéhyde issu de l'étape E2), et produisant au moins un effluent riche en éthanol, un effluent riche en acétaldéhyde et un effluent riche en eau ;
E2) une étape d'élimination des impuretés et des huiles brunes, alimentée au moins par l'effluent éthanol/acétaldéhyde/eau issu de l'étape D1), et par l'effluent riche en eau issu de l'étape E1), et produisant au moins un raffinat eau / éthanol / acétaldéhyde, un effluent huiles brunes légères et un effluent huiles brunes lourdes ;
F) une étape de lavage à l'eau, alimentée par l'effluent sous-produits gazeux issu de l'étape D1), ainsi que par une fraction de l'effluent riche en eau issu de ladite étape E1) et produisant au moins un effluent eau alcoolisée.

2. Procédé selon la revendication 1 dans lequel une étape C2) de traitement final de l'hydrogène est réalisée à l'issue de l'étape C1), ladite étape C2) comprenant au moins d'une section de lavage gaz-liquide, alimentée par l'effluent hydrogène purifié issu de C1), et par un effluent eau pure d'origine externe au procédé ou par un effluent riche en eau issu de l'étape E1), et produisant un effluent hydrogène purifié et un effluent eau usé.

3. Procédé selon l'une des revendications 1 ou 2 dans lequel l'effluent butadiène pré-purifié issu de l'étape D2) subit une étape D2bis) de seconde purification du butadiène avant d'être alimentée dans l'étape D3) de purification ultérieure du butadiène, ladite étape D2bis) comprenant au moins une section de lavage alimentée en fond par ledit effluent butadiène pré-purifié issu de D2), et en tête par une solution absorbante.

4. Procédé selon la revendication 3 dans lequel ladite solution absorbante est une solution aqueuse ayant un pH supérieur à 10, ajusté par l'ajout de soude ou de potasse.

5. Procédé selon la revendication 3 dans lequel ladite solution absorbante est une solution aqueuse de bisulfite de sodium ou de potassium dont le pH est compris entre 5 et 8.

6. Procédé selon la revendication 3 dans lequel ladite solution absorbante est une solution aqueuse contenant un composé de la famille des hydrazines.

7. Procédé selon l'une des revendications 1 à 6 dans lequel l'étape E2) comprend au moins une section de lavage/contre-lavage, une section de distillation des huiles brunes légères, et une section de distillation des huiles brunes lourdes, ladite section de lavage/contre-lavage étant alimentée en un point intermédiaire par ledit effluent éthanol/acétaldéhyde/eau issu de l'étape D1), en fond par un effluent hydrocarbures et en tête par une fraction de l'effluent riche en eau issu de l'étape E1), ladite section de distillation des huiles brunes légères étant alimentée par ledit extrait hydrocarbures de lavages et produisant en tant que distillat ledit effluent huiles brunes légères, et un résidu hydrocarbures, ladite section de distillation des huiles lourdes étant alimentée par une fraction comprise entre 5 et 30% du débit total dudit résidu hydrocarbures et produisant un distillat hydrocarbures et, en tant que résidu, ledit effluent huiles brunes lourdes, ledit distillat hydrocarbures et la fraction non traitée dudit résidu hydrocarbures étant mélangés afin de constituer l'effluent hydrocarbures alimentant ladite section de lavage/contre-lavage.

8. Procédé selon l'une des revendications 1 à 7 dans lequel ladite étape A) est également alimentée par au moins une fraction de ladite charge éthanol.

9. Procédé selon l'une des revendications 1 à 8 dans lequel ladite section réactionnelle de ladite étape B) est également alimentée par un flux riche en éthanol issu de l'étape E1).

10. Procédé selon l'une des revendications 1 à 9 dans lequel ladite section réactionnelle de ladite étape B) est également alimentée par un flux externe d'acétaldéhyde.

11. Procédé selon l'une des revendications 1 à 10 dans lequel ladite étape C1) n'est alimentée par aucun autre flux.

12. Procédé selon l'une des revendications 1 à 11 dans lequel l'effluent riche en éthanol issu de l'étape E1) est alimenté dans ladite section de lavage gaz-liquide de ladite étape C1) à une température inférieure à celle de ladite fraction de l'effluent éthanol/acétaldéhyde issu de l'étape A).

13. Procédé selon l'une des revendications 1 à 12 dans lequel ladite étape D3) comprend au moins une section de séchage, une section de distillation cryogénique et une section de séparation butadiène/butènes par extraction liquide-liquide.

14. Procédé selon l'une des revendications 1 à 13 dans lequel ladite étape D3) comprend au moins une distillation et une distillation extractive.

15. Procédé selon l'une des revendications 1 à 14 dans lequel ladite section E1) est également alimentée par une fraction de ladite charge éthanol.

## Patentansprüche

1. Verfahren zur Herstellung von Butadien aus einem Ethanoleinsatz, umfassend mindestens 80 Gew.-% Ethanol, umfassend mindestens:
A) einen Schritt zum Umwandeln des Ethanols in Acetaldehyd, umfassend mindestens einen Reaktionsabschnitt, der mindestens mit einer Fraktion des aus Schritt E1 erhaltenen ethanolreichen Abstroms gespeist wird, der bei einem Druck im Bereich zwischen 0,1 und 1,0 MPa und bei einer Temperatur im Bereich zwischen 200 und 500 °C in Gegenwart eines Katalysators arbeitet, und einen Trennabschnitt zum Trennen des Abstroms des Reaktionsabschnitts in mindestens einen Wasserstoffabstrom in Gasform und einen Ethanol/Acetaldehyd-Abstrom in flüssiger Form,
B) einen Schritt zum Umwandeln in Butadien, umfassend mindestens einen Reaktionsabschnitt, der mit mindestens einer Fraktion des aus Schritt A) erhaltenen Ethanol/Acetaldehyd-Abstroms, mit einem aus Schritt C1) erhaltenen flüssigen ethanolreichen Abstrom, mit einer aus dem Schritt E1) erhaltenen Fraktion des acetaldehydreichen Abstroms gespeist wird, der in Gegenwart eines Katalysators bei eine Temperatur im Bereich zwischen 300 und 400 °C und bei einem Druck im Bereich zwischen 0,1 und 1,0 MPa arbeitet, wobei die Speisestrommengen derart eingestellt werden, dass das Ethanol/Acetaldehyd-Molverhältnis am Eingang des Reaktionsabschnitts im Bereich zwischen 1 und 5 liegt, und einen Trennabschnitt zum Trennen des Abstroms des Reaktionsabschnitts in mindestens einen gasförmigen Abstrom und einen flüssigen Abstrom;
C1) einen Schritt zum Behandeln des Wasserstoffs, umfassend mindestens einen Verdichtungsabschnitt, der den aus Schritt A) erhaltenen Wasserstoffabstrom bei einem Druck im Bereich zwischen 0,1 und 1,0 MPa verdichtet, und einen Gas-Flüssigkeits-Waschabschnitt, der bei einer Temperatur im Bereich zwischen 15 °C und -30 °C mit einer Fraktion des aus Schritt E1) erhaltenen Ethanolabstroms und mit einer Fraktion des aus Schritt A) erhaltenen Ethanol/Acetaldehydabstroms gespeist wird und der bei einer Temperatur im Bereich zwischen 25 und 60 °C mit dem verdichteten Wasserstoffabstrom gespeist wird, und der mindestens einen flüssigen ethanolreichen Abstrom und einen gereinigten Wasserstoffabstrom herstellt;
D1) einen Schritt zum Extrahieren des Butadiens, umfassend mindestens einen Verdichtungsabschnitt, der den aus Schritt B) erhaltenen gasförmigen Abstrom bei einem Druck im Bereich zwischen 0,1 und 1,0 MPa verdichtet, einen Gas-Flüssigkeits-Waschabschnitt, umfassend eine Waschsäule, die am Kopf bei einer Temperatur im Bereich zwischen 20 und -20 °C mit einem Ethanolstrom, der aus dem Ethanoleinsatz des Verfahrens und/oder einer aus Schritt E1) erhaltenen Fraktion des Ethanolabstroms besteht, und am Boden mit dem aus Schritt B) erhaltenen gasförmigen Abstrom gespeist und abgekühlt wird, und einen Destillationsabschnitt, der bei einem Druck im Bereich zwischen 0,1 und 1 MPa arbeitet, der mindestens mit dem aus Schritt B) erhaltenen flüssigen Abstrom und mit dem flüssigen Abstrom des Gas-Flüssigkeits-Waschabschnitts gespeist wird, wobei Schritt D1) mindestens einen Abstrom gasförmiger Nebenprodukte, einen unraffinierten Butadienabstrom und einen Ethanol/Acetaldehyd/Wasser-Abstrom herstellt;
D2) einen Schritt zum ersten Reinigen des Butadiens, umfassend mindestens einen Gas-Flüssigkeits-Waschabschnitt, der am Boden mit dem aus Schritt D1) erhaltenen unraffinierten Butadienabstrom und am Kopf mit einem Wasserstrom gespeist wird, der ein Wasserstrom mit einem Ursprung außerhalb des Verfahrens zur Herstellung von Butadien und/oder eine Fraktion des aus Schritt E1) erhaltenen Abstroms sein kann, wobei der Waschabschnitt am Kopf einen vorgereinigten Butadienabstrom und am Boden einen Abstrom aus gebrauchtem Wasser herstellt;
D3) einen anschließenden Schritt zum Reinigen des Butadiens, der mit mindestens dem aus dem Schritt D2) erhaltenen vorgereinigten Butadienabstrom gespeist wird und der mindestens einen gereinigten Butadienabstrom herstellt;
E1) einen Schritt zum Behandeln der Abströme, die mit mindestens dem aus Schritt E2) erhaltenen Wasser/Ethanol/Acetaldehyd-Raffinat gespeist werden und der mindestens einen ethanolreichen Abstrom, einen acetaldehydreichen Abstrom und einen wasserreichen Abstrom herstellt;
E2) einen Schritt zum Entfernen von Verunreinigungen und braunen Ölen, der mit mindestens dem aus Schritt D1) erhaltenen Ethanol/Acetaldehyd/Wasser-Abstrom und mit dem aus Schritt E1) erhaltenen wasserreichen Abstrom gespeist wird, und der mindestens ein Wasser/Ethanol/Acetaldehyd-Raffinat, einen Abstrom brauner Leichtöle und einen Abstrom brauner Schweröle herstellt;
F) einen Schritt zum Waschen mit Wasser der mit dem aus Schritt D1) erhaltenen Abstrom von gasförmigen Nebenprodukten sowie mit einer Fraktion des aus Schritt E1) erhaltenen wasserreichen Abstroms gespeist wird und der mindestens einen Abstrom aus alkoholhaltigem Wasser herstellt.

2. Verfahren nach Anspruch 1, wobei ein Schritt C2) zum abschließenden Behandeln des Wasserstoffs am Ende von Schritt C1) ausgeführt wird, wobei der Schritt C2) mindestens einen Gas-Flüssigkeits-Waschabschnitt umfasst, der mit dem aus C1) erhaltenen gereinigten Wasserstoffabstrom und mit einem Abstrom aus reinem Wasser mit einem Ursprung außerhalb des Verfahrens oder mit einem aus Schritt E1) erhaltenen wasserreichen Abstrom gespeist wird, und der einen gereinigten Wasserstoffabstrom und einen Abstrom aus gebrauchtem Wasser herstellt.

3. Verfahren nach einem der Ansprüche 1 oder 2, wobei der aus Schritt D2) erhaltene vorgereinigte Butadienabstrom einem Schritt D2bis) zum zweiten Reinigen des Butadiens unterzogen wird, bevor er in den Schritt D3) zum anschließenden Reinigen des Butadiens gespeist wird, wobei der Schritt D2bis) mindestens einen Waschabschnitt umfasst, der am Boden mit dem aus Schritt D2) erhaltenen vorgereinigten Butadienabstrom und am Kopf mit einer Absorptionslösung gespeist wird.

4. Verfahren nach Anspruch 3, wobei die Absorptionslösung eine wässrige Lösung mit einem pH-Wert von mehr als 10 ist, die durch Zugabe von Natrium- oder Kaliumhydroxid eingestellt wird.

5. Verfahren nach Anspruch 3, wobei die Absorptionslösung eine wässrige Natrium- oder Kaliumbisulfitlösung ist, deren pH-Wert im Bereich zwischen 5 und 8 liegt.

6. Verfahren nach Anspruch 3, wobei die Absorptionslösung eine wässrige Lösung ist, die eine Verbindung aus der Hydrazin-Familie enthält.

7. Verfahren nach einem der Ansprüche 1 bis 6, wobei Schritt E2) mindestens einen Wasch-/Rückwaschabschnitt, einen Abschnitt zum Destillieren von braunen Leichtölen und einen Abschnitt zum Destillieren von braunen Schwerölen umfasst, wobei der Wasch-/Rückwaschabschnitt an einem Zwischenpunkt mit dem aus Schritt D1) erhaltenen Ethanol/Acetaldehyd/Wasser-Abstrom, am Boden mit einem Kohlenwasserstoffabstrom und am Kopf mit einer Fraktion des aus Schritt E1) erhaltenen wasserreichen Abstroms gespeist wird, wobei der Abschnitt zum Destillieren der braunen Leichtöle mit dem Kohlenwasserstoff-Waschextrakt gespeist wird und als Destillat den Abstrom brauner Leichtöle und einen Kohlenwasserstoff-Rückstand herstellt, wobei der Abschnitt zum Destillieren der Schweröle mit einer Fraktion im Bereich zwischen 5 und 30 % des gesamten Stromvolumens des Kohlenwasserstoff-Rückstands und einem Kohlenwasserstoffdestillat gespeist wird und als Rückstand den Abstrom brauner Schweröle herstellt, wobei das Kohlenwasserstoffdestillat und die nicht behandelte Fraktion des Kohlenwasserstoff-Rückstands gemischt werden, um den Kohlenwasserstoffabstrom zu bilden, der den Wasch-/Rückwaschabschnitt speist.

8. Verfahren nach einem der Ansprüche 1 bis 7, wobei der Schritt A) ebenfalls mit mindestens einer Fraktion des Ethanoleinsatzes gespeist wird.

9. Verfahren nach einem der Ansprüche 1 bis 8, wobei der Reaktionsabschnitt des Schritts B) ebenfalls mit einem aus Schritt E1) erhaltenen ethanolreichen Strom gespeist wird.

10. Verfahren nach einem der Ansprüche 1 bis 9, wobei der Reaktionsabschnitt des Schritts B) ebenfalls mit einem externen Acetaldehydstrom gespeist wird.

11. Verfahren nach einem der Ansprüche 1 bis 10, wobei der Schritt C1) nicht mit einem anderen Strom gespeist wird.

12. Verfahren nach einem der Ansprüche 1 bis 11, wobei der aus Schritt E1) erhaltene ethanolreiche Abstrom in den Gas-Flüssigkeits-Waschabschnitt des Schritts C1) bei einer Temperatur unterhalb der der Fraktion des aus Schritt A) erhaltenen Ethanol/Acetaldehyd-Abstroms gespeist wird.

13. Verfahren nach einem der Ansprüche 1 bis 12, wobei der Schritt D3) mindestens einen Trocknungsabschnitt, einen Tieftemperaturdestillationsabschnitt und einen Abschnitt zur Butadien/Buten-Trennung durch Flüssig-Flüssig-Extraktion umfasst.

14. Verfahren nach einem der Ansprüche 1 bis 13, wobei der Schritt D3) mindestens eine Destillation und eine Extraktivdestillation umfasst.

15. Verfahren nach einem der Ansprüche 1 bis 14, wobei der Abschnitt E1) ebenfalls mit einer Fraktion des Ethanoleinsatzes gespeist wird.

## Claims

1. A process for the production of butadiene from an ethanol feed comprising at least 80% by weight of ethanol, comprising at least:
A) a step for conversion of the ethanol to acetaldehyde, comprising at least one reaction section supplied with at least a fraction of ethanol-rich effluent obtained from step E1), operated at a pressure in the range 0.1 to 1.0 MPa and at a temperature in the range 200°C to 500°C in the presence of a catalyst, and a separation section for separating the effluent from said reaction section into at least one hydrogen effluent in the gaseous form and an ethanol/acetaldehyde effluent in the liquid form;
B) a step for conversion into butadiene, comprising at least one reaction section supplied with at least a fraction of said ethanol/acetaldehyde effluent obtained from step A), with an ethanol-rich liquid obtained from step C1), with a fraction of the acetaldehyde-rich effluent obtained from step E1), operated in the presence of a catalyst, at a temperature in the range 300°C to 400°C, and at a pressure in the range 0.1 to 1.0 MPa, the supply flow rates being regulated such that the ethanol/acetaldehyde molar ratio at the inlet to said reaction section is in the range 1 to 5, and a separation section for separating the effluent from said reaction section into at least one gaseous effluent and a liquid effluent;
C1) a hydrogen treatment step, comprising at least one compression section compressing said hydrogen effluent obtained from step A) to a pressure in the range 0.1 to 1.0 MPa, and a gas-liquid scrubbing section supplied, at a temperature in the range 15°C to -30°C, with a fraction of said ethanol effluent obtained from step E1) and with a fraction of said ethanol/acetaldehyde effluent obtained from step A), and supplied, at a temperature in the range 25°C to 60°C, with said compressed hydrogen effluent, and producing at least one liquid ethanol-rich effluent and a purified hydrogen effluent;
D1) a step for extracting butadiene, comprising at least one compression section compressing said gaseous effluent obtained from step B) to a pressure in the range 0.1 to 1.0 MPa, a gas-liquid scrubbing section comprising a scrubbing column supplied overhead, at a temperature in the range 20°C to -20°C, with an ethanol stream constituted by said ethanol feed for the process and/or a fraction of the ethanol effluent obtained from step E1) and at the bottom with said gaseous effluent obtained from step B) and cooled, and a distillation section operated at a pressure in the range 0.1 to 1 MPa, supplied with at least the liquid effluent obtained from said step B) and with the liquid effluent from said gas-liquid scrubbing section, said step D1) producing at least one effluent of gaseous by-products, an unrefined butadiene effluent and an ethanol/acetaldehyde/water effluent;
D2) a first butadiene purification step comprising at least one gas-liquid scrubbing section the bottom of which is supplied with the unrefined butadiene effluent obtained from D1) and the head of which is supplied with a stream of water which may be a stream of water with an origin external to said butadiene production process and/or a fraction of the water effluent obtained from step E1), said scrubbing section producing a pre-purified butadiene effluent overhead and a spent water effluent from the bottom;
D3) a subsequent butadiene purification step supplied with at least said pre-purified butadiene effluent obtained from said step D2), and producing at least one purified butadiene effluent;
E1) an effluent treatment step supplied with at least the water/ethanol/acetaldehyde raffinate obtained from step E2), and producing at least one ethanol-rich effluent, an acetaldehyde-rich effluent and a water-rich effluent;
E2) a step for eliminating impurities and brown oils, supplied with at least the ethanol/acetaldehyde/water effluent obtained from step D1), and with the water-rich effluent obtained from step E1), and producing at least one water/ethanol/acetaldehyde raffinate, a light brown oil effluent and a heavy brown oil effluent;
F) a step for scrubbing with water supplied by the effluent of gaseous by-products obtained from step D1), as well as with a fraction of the water-rich effluent obtained from said step E1) and producing at least one alcohol-containing water effluent.

2. The process according to claim 1, wherein a final hydrogen treatment step C2) is carried out at the end of step C1), said step C2) comprising at least one gas-liquid scrubbing section supplied with the purified hydrogen effluent obtained from C1) and with a pure water effluent originating from outside the process or with a water-rich effluent obtained from step E1), and producing a purified hydrogen effluent and a spent water effluent.

3. The process according to claim 1 or claim 2, wherein the pre-purified butadiene effluent obtained from step D2) undergoes a second butadiene purification step D2bis) before being supplied to the subsequent butadiene purification step D3), said step D2bis) comprising at least one scrubbing section the bottom of which is supplied with said pre-purified butadiene effluent obtained from D2), and the head of which is supplied with an absorbent solution.

4. The process according to claim 3, wherein said absorbent solution is an aqueous solution with a pH of more than 10, adjusted by adding sodium or potassium hydroxide.

5. The process according to claim 3, wherein said absorbent solution is an aqueous sodium or potassium bisulphite solution the pH of which is in the range 5 to 8.

6. The process according to claim 3, wherein said absorbent solution is an aqueous solution containing a compound from the hydrazine family.

7. The process according to one of claims 1 to 6, wherein step E2) comprises at least one scrubbing/back-scrubbing section, a section for the distillation of light brown oils, and a section for the distillation of heavy brown oils, said scrubbing/back-scrubbing section being supplied at an intermediate point with said ethanot/acetaldehyde/water effluent obtained from step D1), at the bottom with a hydrocarbon effluent and overhead with a fraction of the water-rich effluent obtained from step E1), said light brown oils distillation section being supplied with said scrubbing hydrocarbon extract and producing, as a distillate, said light brown oil effluent and a hydrocarbon residue, said heavy oils distillation section being supplied with a fraction in the range 5% to 30% of the total flow rate of said hydrocarbon residue and producing a hydrocarbon distillate and, as a residue, said heavy brown oil effluent, said hydrocarbon distillate and the untreated fraction of said hydrocarbon residue being mixed to constitute the hydrocarbon effluent supplying said scrubbing/back-scrubbing section.

8. The process according to one of claims 1 to 7, wherein said step A) is further supplied with at least one fraction of said ethanol feed.

9. The process according to one of claims 1 to 8, wherein said reaction section of said step B) is further supplied with an ethanol-rich stream obtained from step E1).

10. The process according to one of claims 1 to 9, wherein said reaction section of said step B) is further supplied with an external acetaldehyde stream.

11. The process according to one of claims 1 to 10, wherein said step C1) is not supplied with any other stream.

12. The process according to one of claims 1 to 11, wherein the ethanol-rich effluent obtained from step E1) is supplied to said gas-liquid scrubbing section of said step C1) at a temperature which is below to the one of said ethanol/acctaldehyde effluent obtained from step A).

13. The process according to one of claims 1 to 12, wherein said step D3) comprises at least one drying section, a cryogenic distillation section and a section for butadiene/butenes separation by liquid-liquid extraction.

14. The process according to one of claims 1 to 13, wherein said step D3) comprises at least one distillation and an extractive distillation.

15. The process according to one of claims 1 to 14, wherein said section E1) is further supplied with a fraction of said ethanol feed.
